# EUROPEAN PATENT APPLICATION

(11) **EP 4 057 009 A1**
(43) Date of publication of application: **14.09.2022**
(21) Application number: 22160377.2
(22) Date of filing: 07.03.2022
(51) Int. Cl.: G01N 33/68, G01N 33/543, C07K 14/415

(54) **METHODS AND REAGENTS FOR DETECTING AN ANTIBODY TO A SESAME NSLTP**

(30) Priority: 08.03.2021 EP 21161331
(71) Applicant: Euroimmun Medizinische Labordiagnostika AG, 23560 Lübeck (DE)
(72) Inventor: WEIMANN, Yvonne, 23560 Lübeck (DE); SUER, Waltraud, 23911 Buchholz (DE); WIERZBA, Eva, 23896 Nusse (DE); BRIX, Bettina, 23562 Lübeck (DE)

(57) **Abstract**

The present invention relates to a polypeptide comprising a sesame nsLTP characterized by SEQ ID NO11 or a variant thereof, wherein the polypeptide is isolated, purified and/or immobilized, a diagnostically useful carrier comprising a solid phase coated with the polypeptide, a pharmaceutical composition comprising the polypeptide, a method comprising the step detecting in a sample the presence or absence of an antibody binding specifically to a sesame nsLTP characterized by SEQ ID NO11, a use of a polypeptide comprising a sesame nsLTP characterized by SEQ ID NO11 or a variant thereof, the polypeptide, carrier or kit for detecting an antibody binding specifically to a sesame nsLTP characterized by SEQ ID NO11 or for manufacturing a device for detecting said antibody, an enriched, isolated or purified antibody, preferably IgG or IgE class antibody, or a recombinant antibody binding specifically to a sesame nsLTP characterized by SEQ ID NO11 and a method for isolating such an antibody.

SEQUENCE LISTING

<110>  EUROIMMUN Medizinische Labordiagnostika AG

<120>  Methods and reagents for detecting an antibody to a sesame nsLTP

<130>  19PP026EP3

<160>  56

<170>  PatentIn version 3.5

<210>  1
<211>  96
<212>  PRT
<213>  Sesamum indicum

<400>  1

Met Lys Lys Gly Ala Ala Ala Val Trp Trp Val Val Ala Ile Val Val
1               5                   10                  15

Val Leu Ala Val Ala Glu Val His Glu Thr Gly Ala Ala Pro Cys Asn
            20                  25                  30

Pro Val Glu Leu Ser Pro Cys Leu Ala Ala Ile Ser Gly Ser Gln Pro
        35                  40                  45

Pro Ser Ala Glu Cys Cys Ser Lys Leu Lys Glu Gln Val Pro Cys Phe
    50                  55                  60

Cys Asp Tyr Val Lys Asn Pro Val Phe Lys Pro Tyr Val Asp Ser Pro
65                  70                  75                  80

Asn Ala Lys Lys Val Ala Ala Thr Cys Gly Val Ala Phe Pro Thr Cys
                85                  90                  95

<210>  2
<211>  119
<212>  PRT
<213>  Sesamum indicum

<400>  2

Met Glu Lys Ala Thr Cys Leu Val Leu Ala Val Thr Met Val Val Val
1               5                   10                  15

Ala Leu Ala Pro Pro Gln Ala Glu Ala Ala Ile Ser Cys Gly Thr Val
            20                  25                  30

Ala Asn Asp Leu Ser Pro Cys Ile Asn Tyr Val Met Tyr Gly Gly Ala
        35                  40                  45

Gly Thr Pro Pro Val Pro Cys Cys Thr Gly Ile Lys Thr Leu Tyr Asn

50                  55                  60

Gln Ala Ser Ser Thr Ala Asp Arg Gln Ala Val Cys Gly Cys Leu Lys
65                  70                  75                  80

Ser Val Ala Gly Gln Ala Ser Pro Ala Ile Ile Gly Thr Ala Ala Ala
                85                  90                  95

Leu Pro Gly Lys Cys Gly Val Ser Ile Pro Tyr Glu Ile Ser Pro Ser
            100                 105                 110

Thr Asp Cys Ser Lys Val His
        115

<210>  3
<211>  118
<212>  PRT
<213>  Sesamum indicum

<400>  3

Met Ala Gly Thr Ile Lys Pro Met Phe Val Val Leu Ile Ala Val Val
1               5                   10                  15

Ser Ile Met Ser Val Val Pro Glu Gly Glu Ala Ala Ile Gly Cys Gly
            20                  25                  30

Thr Val Val Ser Tyr Leu Asn Ser Cys Phe Pro Tyr Val Thr Gly Thr
        35                  40                  45

Gly Ala Leu Gly Gly Cys Cys Lys Gly Ile Lys Asp Leu Tyr Ala Ala
    50                  55                  60

Ala Gln Thr Thr Ala Asp Arg Gln Ser Val Cys Asp Cys Leu Lys Asp
65                  70                  75                  80

Leu Ala Ala Ser Thr Thr Gly Val Asp Leu Gly Lys Ala Ala Gly Leu
                85                  90                  95

Pro Ser Gln Cys Gly Leu Asn Ile Pro Tyr Lys Ile Ile Pro Ser Thr
            100                 105                 110

Asp Cys Ser Lys Val Gln
        115

<210>  4
<211>  118
<212>  PRT
<213>  Sesamum indicum

<400>  4

Met Ala Gly Ile Ile Arg Pro Met Cys Ile Leu Leu Ile Ala Ala Val
1               5                   10                  15

Leu Ala Met Ala Val Ala Pro Arg Gly Glu Ala Ala Ile Gly Cys Gly
            20                  25                  30

Thr Val Val Ser Tyr Leu Asn Pro Cys Leu Pro Tyr Val Thr Asn Lys
        35                  40                  45

Gly Pro Leu Gly Ser Cys Cys Gly Gly Val Lys Gly Leu Tyr Gly Ala
    50                  55                  60

Ala Gln Thr Ala Gln Asp Arg Gln Ser Val Cys Ser Cys Leu Lys Ser
65                  70                  75                  80

Leu Ala Gly Ser Tyr Lys Asp Val Asp Leu Asn Lys Ala Ala Gly Leu
                85                  90                  95

Pro Gly Gln Cys Gly Val Asn Ile Pro Tyr Lys Ile Ser Pro Ser Thr
            100                 105                 110

Asp Cys Ser Lys Val Asn
        115


<210>  5
<211>  118
<212>  PRT
<213>  Sesamum indicum

<400>  5

Met Ala Ser Met Ile Lys Ala Leu Cys Val Ala Leu Val Gly Ala Val
1               5                   10                  15

Leu Val Ile Ala Val Val Pro Pro Ala Glu Ala Ala Leu Gly Cys Gly
            20                  25                  30

Ser Val Ile Ser Tyr Leu Gly Ser Cys Arg Pro Tyr Val Thr Asp Lys
        35                  40                  45

Gly Pro Leu Gly Gly Cys Cys Ser Gly Val Lys Gly Leu Tyr Lys Ala
    50                  55                  60

Ala Lys Thr Thr Ala Asp Arg Gln Ala Thr Cys Ser Cys Leu Lys Thr
65                  70                  75                  80

Leu Ala Ser Thr Tyr Lys Gly Val Asn Leu Ser Lys Ala Ala Gly Leu
                85                  90                  95

Pro Gln Gln Cys Gly Val Asn Ile Pro Tyr Lys Ile Ser Pro Ser Thr
            100                 105                 110

Asp Cys Ser Lys Val Thr
        115

<210>  6
<211>  236
<212>  DNA
<213>  Artificial Sequence

<220>
<223>  Sesamum indicum lipid transfer protein (LTP1), cDNA fragment

<400>  6
agcggtctca catggcgcct tgcaacccgg tagagctgtc gccctgcctt gcagcgatat      60

cagggtcgca gccgccgtcg gcggagtgct gcagcaagct gaaggagcag gtgccctgct     120

tctgtgacta cgtgaagaac ccggttttca agccgtatgt cgactctcct aatgccaaga     180

aggttgctgc aacctgcggc gtcgccttcc ccacatgttg actcgaggag accgaa         236

<210>  7
<211>  316
<212>  DNA
<213>  Artificial Sequence

<220>
<223>  Sesamum indicum lipid transfer protein (LTP2), cDNA fragment

<400>  7
agcgaagaca tcatggccat atcatgcggc actgtggcca acgacctgtc cccgtgcatc      60

aactacgtga tgtatggtgg ggcggggacg ccaccggtac catgctgcac cgggataaag     120

accctctaca atcaggcaag cagcaccgct gaccgccagg ctgtttgtgg gtgcttgaaa     180

tctgttgccg gccaagccag ccctgccatc attggcacag ccgccgcact tcctggcaaa     240

tgtggcgtct ctattcctta cgagatcagc ccctccacgg attgctccaa ggttcattaa     300

ctcgagagtc ttcggt                                                     316

<210>  8
<211>  305
<212>  DNA
<213>  Artificial Sequence

<220>
<223>  Sesamum indicum lipid transfer protein (LTP3), cDNA fragment

<400>  8
ttcggtctca catggcgata ggttgcggca ctgtagtttc atatctgaat tcatgctttc      60

catacgtgac cggaactggt gcgttgggtg gatgctgtaa agggattaag gatctctatg     120

cggcggccca aaccacagcc gaccgacaga gcgtgtgcga ctgcctgaaa gatcttgctg 180

cctcgaccac tggcgtcgac ctcggcaaag ctgctggact ccccagccag tgtggtctta 240

acatccccta caagatcatt ccttcaactg attgctcaaa ggtgcaatga ctcgaggaga 300

ccacc 305

<210> 9
<211> 307
<212> DNA
<213> Artificial Sequence

<220>
<223> Sesamum indicum lipid transfer protein (LTP4), cDNA fragment

<400> 9
accgaagaca tcatggcaat aggctgcggc acggtggttt catacctgaa tccatgcctg 60

ccatatgtaa ccaacaaagg ccctctaggc agttgttgcg gtggagttaa gggtctgtat 120

ggagcagccc agactgcaca agatcgtcaa agcgtgtgca gctgcttgaa atctctcgct 180

ggctcataca aagatgtcga cctgaacaag gccgctggac tccctggaca atgtggcgtc 240

aacattccct acaagattag cccttccact gattgctcaa aggtgaattg actcgagagt 300

cttcacc 307

<210> 10
<211> 307
<212> DNA
<213> Artificial Sequence

<220>
<223> Sesamum indicum lipid transfer protein (LTP5), cDNA fragment

<400> 10
agcgaagaca tcatggcatt aggctgcggc agcgtgatat cttatctggg ttcatgccgc 60

ccctatgtga ccgacaaagg tcctttaggt ggttgctgca gtggggtgaa gggactctat 120

aaggctgcaa aaaccaccgc ggatcgtcag gccacttgca gctgcctcaa aactcttgcc 180

agtacctaca aaggtgtcaa tcttagcaag gcagccggac ttcctcagca atgtggtgtt 240

aacattccct ataaaatcag cccttccacc gattgctcaa aggtgacgtg actcgagagt 300

cttcacc 307

<210> 11
<211> 77
<212> PRT
<213> Artificial Sequence

<220>
<223> consensus sequence 1

<220>

<221>  misc_feature
<222>  (1)..(2)
<223>  Xaa can be any naturally occurring amino acid

<220>
<221>  misc_feature
<222>  (5)..(9)
<223>  Xaa can be any naturally occurring amino acid

<220>
<221>  misc_feature
<222>  (11)..(12)
<223>  Xaa can be any naturally occurring amino acid

<220>
<221>  misc_feature
<222>  (14)..(28)
<223>  Xaa can be any naturally occurring amino acid

<220>
<221>  misc_feature
<222>  (31)..(33)
<223>  Xaa can be any naturally occurring amino acid

<220>
<221>  misc_feature
<222>  (35)..(49)
<223>  Xaa can be any naturally occurring amino acid

<220>
<221>  misc_feature
<222>  (51)..(51)
<223>  Xaa can be any naturally occurring amino acid

<220>
<221>  misc_feature
<222>  (53)..(75)
<223>  Xaa can be any naturally occurring amino acid

<400>  11

Xaa Xaa Cys Gly Xaa Xaa Xaa Xaa Xaa Leu Xaa Xaa Cys Xaa Xaa Xaa
1               5                   10                  15

Xaa Xaa Xaa Xaa Xaa Xaa Xaa Xaa Xaa Xaa Xaa Xaa Cys Cys Xaa Xaa
            20                  25                  30

Xaa Lys Xaa Xaa Xaa Xaa Xaa Xaa Xaa Xaa Xaa Xaa Xaa Xaa Xaa Xaa
        35                  40                  45

Xaa Cys Xaa Cys Xaa Xaa Xaa Xaa Xaa Xaa Xaa Xaa Xaa Xaa Xaa Xaa
    50                  55                  60

Xaa Xaa Xaa Xaa Xaa Xaa Xaa Xaa Xaa Xaa Xaa Cys Gly
65                  70                  75

<210>  12
<211>  93

<212>  PRT
<213>  Artificial Sequence

<220>
<223>  consensus sequence 2



<220>
<221>  misc_feature
<222>  (1)..(2)
<223>  Xaa can be any naturally occurring amino acid

<220>
<221>  misc_feature
<222>  (5)..(5)
<223>  Xaa can be any naturally occurring amino acid

<220>
<221>  misc_feature
<222>  (7)..(9)
<223>  Xaa can be any naturally occurring amino acid

<220>
<221>  misc_feature
<222>  (11)..(12)
<223>  Xaa can be any naturally occurring amino acid

<220>
<221>  misc_feature
<222>  (14)..(15)
<223>  Xaa can be any naturally occurring amino acid

<220>
<221>  misc_feature
<222>  (18)..(20)
<223>  Xaa can be any naturally occurring amino acid

<220>
<221>  misc_feature
<222>  (22)..(28)
<223>  Xaa can be any naturally occurring amino acid

<220>
<221>  misc_feature
<222>  (31)..(31)
<223>  Xaa can be any naturally occurring amino acid

<220>
<221>  misc_feature
<222>  (33)..(33)
<223>  Xaa can be any naturally occurring amino acid

<220>
<221>  misc_feature
<222>  (35)..(35)
<223>  Xaa can be any naturally occurring amino acid

<220>
<221>  misc_feature
<222>  (38)..(39)
<223>  Xaa can be any naturally occurring amino acid

<220>

<221>  misc_feature
<222>  (41)..(44)
<223>  Xaa can be any naturally occurring amino acid

<220>
<221>  misc_feature
<222>  (48)..(49)
<223>  Xaa can be any naturally occurring amino acid

<220>
<221>  misc_feature
<222>  (51)..(51)
<223>  Xaa can be any naturally occurring amino acid

<220>
<221>  misc_feature
<222>  (55)..(56)
<223>  Xaa can be any naturally occurring amino acid

<220>
<221>  misc_feature
<222>  (58)..(67)
<223>  Xaa can be any naturally occurring amino acid

<220>
<221>  misc_feature
<222>  (70)..(70)
<223>  Xaa can be any naturally occurring amino acid

<220>
<221>  misc_feature
<222>  (73)..(74)
<223>  Xaa can be any naturally occurring amino acid

<220>
<221>  misc_feature
<222>  (77)..(78)
<223>  Xaa can be any naturally occurring amino acid

<220>
<221>  misc_feature
<222>  (82)..(82)
<223>  Xaa can be any naturally occurring amino acid

<220>
<221>  misc_feature
<222>  (84)..(84)
<223>  Xaa can be any naturally occurring amino acid

<220>
<221>  misc_feature
<222>  (93)..(93)
<223>  Xaa can be any naturally occurring amino acid

<400>  12

Xaa Xaa Cys Gly Xaa Val Xaa Xaa Xaa Leu Xaa Xaa Cys Xaa Xaa Tyr
1               5                   10                  15

Val Xaa Xaa Xaa Gly Xaa Xaa Xaa Xaa Xaa Xaa Xaa Cys Cys Xaa Gly
            20                  25                  30

Xaa Lys Xaa Leu Tyr Xaa Xaa Ala Xaa Xaa Xaa Xaa Asp Arg Gln Xaa
        35                  40                  45

Xaa Cys Xaa Cys Leu Lys Xaa Xaa Ala Xaa Xaa Xaa Xaa Xaa Xaa Xaa
    50                  55                  60

Xaa Xaa Xaa Ala Ala Xaa Leu Pro Xaa Xaa Cys Gly Xaa Xaa Ile Pro
65                  70                  75                  80

Tyr Xaa Ile Xaa Pro Ser Thr Asp Cys Ser Lys Val Xaa
                85                  90

<210>  13
<211>  89
<212>  PRT
<213>  Artificial Sequence

<220>
<223>  consensus sequence 3

<220>
<221>  misc_feature
<222>  (1)..(1)
<223>  Xaa can be any naturally occurring amino acid

<220>
<221>  misc_feature
<222>  (5)..(5)
<223>  Xaa can be any naturally occurring amino acid

<220>
<221>  misc_feature
<222>  (7)..(7)
<223>  Xaa can be any naturally occurring amino acid

<220>
<221>  misc_feature
<222>  (11)..(12)
<223>  Xaa can be any naturally occurring amino acid

<220>
<221>  misc_feature
<222>  (14)..(14)
<223>  Xaa can be any naturally occurring amino acid

<220>
<221>  misc_feature
<222>  (19)..(20)
<223>  Xaa can be any naturally occurring amino acid

<220>
<221>  misc_feature
<222>  (22)..(22)
<223>  Xaa can be any naturally occurring amino acid

<220>
<221>  misc_feature

<222>  (25)..(25)
<223>  Xaa can be any naturally occurring amino acid

<220>
<221>  misc_feature
<222>  (28)..(28)
<223>  Xaa can be any naturally occurring amino acid

<220>
<221>  misc_feature
<222>  (30)..(30)
<223>  Xaa can be any naturally occurring amino acid

<220>
<221>  misc_feature
<222>  (32)..(32)
<223>  Xaa can be any naturally occurring amino acid

<220>
<221>  misc_feature
<222>  (35)..(35)
<223>  Xaa can be any naturally occurring amino acid

<220>
<221>  misc_feature
<222>  (38)..(38)
<223>  Xaa can be any naturally occurring amino acid

<220>
<221>  misc_feature
<222>  (40)..(41)
<223>  Xaa can be any naturally occurring amino acid

<220>
<221>  misc_feature
<222>  (45)..(46)
<223>  Xaa can be any naturally occurring amino acid

<220>
<221>  misc_feature
<222>  (48)..(48)
<223>  Xaa can be any naturally occurring amino acid

<220>
<221>  misc_feature
<222>  (52)..(52)
<223>  Xaa can be any naturally occurring amino acid

<220>
<221>  misc_feature
<222>  (55)..(59)
<223>  Xaa can be any naturally occurring amino acid

<220>
<221>  misc_feature
<222>  (61)..(61)
<223>  Xaa can be any naturally occurring amino acid

<220>
<221>  misc_feature
<222>  (63)..(63)
<223>  Xaa can be any naturally occurring amino acid

<220>
<221>  misc_feature
<222>  (70)..(70)
<223>  Xaa can be any naturally occurring amino acid

<220>
<221>  misc_feature
<222>  (74)..(74)
<223>  Xaa can be any naturally occurring amino acid

<220>
<221>  misc_feature
<222>  (81)..(81)
<223>  Xaa can be any naturally occurring amino acid

<400>  13

Xaa Gly Cys Gly Xaa Val Xaa Ser Tyr Leu Xaa Xaa Cys Xaa Pro Tyr
1               5                   10                  15

Val Thr Xaa Xaa Gly Xaa Leu Gly Xaa Cys Cys Xaa Gly Xaa Lys Xaa
            20                  25                  30

Leu Tyr Xaa Ala Ala Xaa Thr Xaa Xaa Asp Arg Gly Xaa Xaa Cys Xaa
        35                  40                  45

Cys Leu Lys Xaa Leu Ala Xaa Xaa Xaa Xaa Xaa Val Xaa Leu Xaa Lys
    50                  55                  60

Ala Ala Gly Leu Pro Xaa Gln Cys Gly Xaa Asn Ile Pro Tyr Lys Ile
65                  70                  75                  80

Xaa Pro Ser Thr Asp Cys Ser Lys Val
                85

<210>  14
<211>  0
<212>  PRT
<213>  Sesamum indicum

<400>  14
000
<210>  15
<211>  0
<212>  PRT
<213>  Sesamum indicum

<400>  15
000
<210>  16
<211>  0
<212>  PRT
<213>  Sesamum indicum

<400>  16
000
<210>  17

<211>  185
<212>  PRT
<213>  Artificial Sequence

<220>
<223>  alpha-factor-sp-H6-linker-PSc-Ses iLTP1

<400>  17

Met Arg Phe Pro Ser Ile Phe Thr Ala Val Leu Phe Ala Ala Ser Ser
1               5                   10                  15

Ala Leu Ala Ala Pro Val Asn Thr Thr Thr Glu Asp Glu Thr Ala Gln
            20                  25                  30

Ile Pro Ala Glu Ala Val Ile Gly Tyr Ser Asp Leu Glu Gly Asp Phe
        35                  40                  45

Asp Val Ala Val Leu Pro Phe Ser Asn Ser Thr Asn Asn Gly Leu Leu
    50                  55                  60

Phe Ile Asn Thr Thr Ile Ala Ser Ile Ala Ala Lys Glu Glu Gly Val
65                  70                  75                  80

Ser Leu Glu Lys Arg Ala Met Ala His His His His His His Gly Gly
                85                  90                  95

Gly Ser Gly Gly Gly Ser Gly Gly Gly Ser Leu Glu Val Leu Phe Gln
            100                 105                 110

Gly Pro Ser Ala Met Ala Pro Cys Asn Pro Val Glu Leu Ser Pro Cys
        115                 120                 125

Leu Ala Ala Ile Ser Gly Ser Gln Pro Pro Ser Ala Glu Cys Cys Ser
    130                 135                 140

Lys Leu Lys Glu Gln Val Pro Cys Phe Cys Asp Tyr Val Lys Asn Pro
145                 150                 155                 160

Val Phe Lys Pro Tyr Val Asp Ser Pro Asn Ala Lys Lys Val Ala Ala
                165                 170                 175

Thr Cys Gly Val Ala Phe Pro Thr Cys
            180                 185


<210>  18
<211>  211
<212>  PRT
<213>  Artificial Sequence

<220>

<223>  alpha-factor-sp-H6-linker-PSc-Ses iLTP2

<400>  18

Met Arg Phe Pro Ser Ile Phe Thr Ala Val Leu Phe Ala Ala Ser Ser
1               5                   10                  15

Ala Leu Ala Ala Pro Val Asn Thr Thr Thr Glu Asp Glu Thr Ala Gln
            20                  25                  30

Ile Pro Ala Glu Ala Val Ile Gly Tyr Ser Asp Leu Glu Gly Asp Phe
        35                  40                  45

Asp Val Ala Val Leu Pro Phe Ser Asn Ser Thr Asn Asn Gly Leu Leu
    50                  55                  60

Phe Ile Asn Thr Thr Ile Ala Ser Ile Ala Ala Lys Glu Glu Gly Val
65                  70                  75                  80

Ser Leu Glu Lys Arg Ala Met Ala His His His His His His Gly Gly
                85                  90                  95

Gly Ser Gly Gly Gly Ser Gly Gly Gly Ser Leu Glu Val Leu Phe Gln
            100                 105                 110

Gly Pro Ser Ala Met Ala Ile Ser Cys Gly Thr Val Ala Asn Asp Leu
        115                 120                 125

Ser Pro Cys Ile Asn Tyr Val Met Tyr Gly Gly Ala Gly Thr Pro Pro
    130                 135                 140

Val Pro Cys Cys Thr Gly Ile Lys Thr Leu Tyr Asn Gln Ala Ser Ser
145                 150                 155                 160

Thr Ala Asp Arg Gln Ala Val Cys Gly Cys Leu Lys Ser Val Ala Gly
                165                 170                 175

Gln Ala Ser Pro Ala Ile Ile Gly Thr Ala Ala Ala Leu Pro Gly Lys
            180                 185                 190

Cys Gly Val Ser Ile Pro Tyr Glu Ile Ser Pro Ser Thr Asp Cys Ser
        195                 200                 205

Lys Val His
    210

<210>  19
<211>  208
<212>  PRT

<213>  Artificial Sequence

<220>
<223>  alpha-factor-sp-H6-linker-PSc-Ses iLTP3

<400>  19

Met Arg Phe Pro Ser Ile Phe Thr Ala Val Leu Phe Ala Ala Ser Ser
1               5                   10                  15

Ala Leu Ala Ala Pro Val Asn Thr Thr Thr Glu Asp Glu Thr Ala Gln
            20                  25                  30

Ile Pro Ala Glu Ala Val Ile Gly Tyr Ser Asp Leu Glu Gly Asp Phe
        35                  40                  45

Asp Val Ala Val Leu Pro Phe Ser Asn Ser Thr Asn Asn Gly Leu Leu
    50                  55                  60

Phe Ile Asn Thr Thr Ile Ala Ser Ile Ala Ala Lys Glu Glu Gly Val
65                  70                  75                  80

Ser Leu Glu Lys Arg Ala Met Ala His His His His His His Gly Gly
                85                  90                  95

Gly Ser Gly Gly Gly Ser Gly Gly Gly Ser Leu Glu Val Leu Phe Gln
            100                 105                 110

Gly Pro Ser Ala Met Ala Ile Gly Cys Gly Thr Val Val Ser Tyr Leu
        115                 120                 125

Asn Ser Cys Phe Pro Tyr Val Thr Gly Thr Gly Ala Leu Gly Gly Cys
    130                 135                 140

Cys Lys Gly Ile Lys Asp Leu Tyr Ala Ala Ala Gln Thr Thr Ala Asp
145                 150                 155                 160

Arg Gln Ser Val Cys Asp Cys Leu Lys Asp Leu Ala Ala Ser Thr Thr
                165                 170                 175

Gly Val Asp Leu Gly Lys Ala Ala Gly Leu Pro Ser Gln Cys Gly Leu
            180                 185                 190

Asn Ile Pro Tyr Lys Ile Ile Pro Ser Thr Asp Cys Ser Lys Val Gln
        195                 200                 205

<210>  20
<211>  208
<212>  PRT
<213>  Artificial Sequence

<220>
<223>  alpha-factor-sp-H6-linker-PSc-Ses iLTP4

<400>  20

Met Arg Phe Pro Ser Ile Phe Thr Ala Val Leu Phe Ala Ala Ser Ser
1               5                   10                  15

Ala Leu Ala Ala Pro Val Asn Thr Thr Thr Glu Asp Glu Thr Ala Gln
            20                  25                  30

Ile Pro Ala Glu Ala Val Ile Gly Tyr Ser Asp Leu Glu Gly Asp Phe
        35                  40                  45

Asp Val Ala Val Leu Pro Phe Ser Asn Ser Thr Asn Asn Gly Leu Leu
    50                  55                  60

Phe Ile Asn Thr Thr Ile Ala Ser Ile Ala Ala Lys Glu Glu Gly Val
65                  70                  75                  80

Ser Leu Glu Lys Arg Ala Met Ala His His His His His His Gly Gly
                85                  90                  95

Gly Ser Gly Gly Gly Ser Gly Gly Gly Ser Leu Glu Val Leu Phe Gln
            100                 105                 110

Gly Pro Ser Ala Met Ala Ile Gly Cys Gly Thr Val Val Ser Tyr Leu
        115                 120                 125

Asn Pro Cys Leu Pro Tyr Val Thr Asn Lys Gly Pro Leu Gly Ser Cys
    130                 135                 140

Cys Gly Gly Val Lys Gly Leu Tyr Gly Ala Ala Gln Thr Ala Gln Asp
145                 150                 155                 160

Arg Gln Ser Val Cys Ser Cys Leu Lys Ser Leu Ala Gly Ser Tyr Lys
                165                 170                 175

Asp Val Asp Leu Asn Lys Ala Ala Gly Leu Pro Gly Gln Cys Gly Val
            180                 185                 190

Asn Ile Pro Tyr Lys Ile Ser Pro Ser Thr Asp Cys Ser Lys Val Asn
        195                 200                 205

<210>  21
<211>  208
<212>  PRT
<213>  Artificial Sequence

<220>
<223>  alpha-factor-sp-H6-linker-PSc-Ses iLTP5

<400>  21

Met Arg Phe Pro Ser Ile Phe Thr Ala Val Leu Phe Ala Ala Ser Ser
1               5                   10                  15

Ala Leu Ala Ala Pro Val Asn Thr Thr Thr Glu Asp Glu Thr Ala Gln
            20                  25                  30

Ile Pro Ala Glu Ala Val Ile Gly Tyr Ser Asp Leu Glu Gly Asp Phe
        35                  40                  45

Asp Val Ala Val Leu Pro Phe Ser Asn Ser Thr Asn Asn Gly Leu Leu
    50                  55                  60

Phe Ile Asn Thr Thr Ile Ala Ser Ile Ala Ala Lys Glu Glu Gly Val
65                  70                  75                  80

Ser Leu Glu Lys Arg Ala Met Ala His His His His His His Gly Gly
                85                  90                  95

Gly Ser Gly Gly Gly Ser Gly Gly Gly Ser Leu Glu Val Leu Phe Gln
            100                 105                 110

Gly Pro Ser Ala Met Ala Leu Gly Cys Gly Ser Val Ile Ser Tyr Leu
        115                 120                 125

Gly Ser Cys Arg Pro Tyr Val Thr Asp Lys Gly Pro Leu Gly Gly Cys
    130                 135                 140

Cys Ser Gly Val Lys Gly Leu Tyr Lys Ala Ala Lys Thr Thr Ala Asp
145                 150                 155                 160

Arg Gln Ala Thr Cys Ser Cys Leu Lys Thr Leu Ala Ser Thr Tyr Lys
                165                 170                 175

Gly Val Asn Leu Ser Lys Ala Ala Gly Leu Pro Gln Gln Cys Gly Val
            180                 185                 190

Asn Ile Pro Tyr Lys Ile Ser Pro Ser Thr Asp Cys Ser Lys Val Thr
        195                 200                 205

<210>  22
<211>  3778
<212>  DNA
<213>  Artificial Sequence

<220>

<223>  pPICZd-sp-N-(PSc)-mSes iLTP1

<400>  22
atcatcatta ttagcttact ttcataattg cgactggttc caattgacaa gcttttgatt      60

ttaacgactt ttaacgacaa cttgagaaga tcaaaaaaca actaattatt cgaaacgatg     120

agatttcctt caatttttac tgctgtttta ttcgcagcat cctccgcatt agctgctcca     180

gtcaacacta caacagaaga tgaaacggca caaattccgg ctgaagctgt catcggttac     240

tcagatttag aaggggattt cgatgttgct gttttgccat tttccaacag cacaaataac     300

gggttattgt ttataaatac tactattgcc agcattgctg ctaaagaaga aggggtatct     360

ttagagaaaa gagccatggc tcatcatcat catcatcatg gcggtggaag tggaggaggt     420

agtggaggcg ggtcacttga agtcttgttt caaggtccat ctgccatggc gccttgcaac     480

ccggtagagc tgtcgccctg ccttgcagcg atatcagggt cgcagccgcc gtcggcggag     540

tgctgcagca agctgaagga gcaggtgccc tgcttctgtg actacgtgaa gaacccggtt     600

ttcaagccgt atgtcgactc tcctaatgcc aagaaggttg ctgcaacctg cggcgtcgcc     660

ttccccacat gttgactcga ccatcatcat catcatcatt gagtttgtag ccttagacat     720

gactgttcct cagttcaagt tgggcactta cgagaagacc ggtcttgcta gattctaatc     780

aagaggatgt cagaatgcca tttgcctgag agatgcaggc ttcatttttg atactttttt     840

atttgtaacc tatatagtat aggatttttt ttgtcatttt gtttcttctc gtacgagctt     900

gctcctgatc agcctatctc gcagctgatg aatatcttgt ggtaggggtt tgggaaaatc     960

attcgagttt gatgtttttc ttggtatttc ccactcctct tcagagtaca gaagattaag    1020

tgagaccttc gtttgtgcgg atcccccaca caccatagct tcaaaatgtt tctactcctt    1080

ttttactctt ccagattttc tcggactccg cgcatcgccg taccacttca aaacacccaa    1140

gcacagcata ctaaatttcc cctctttctt cctctagggt gtcgttaatt acccgtacta    1200

aaggtttgga aaagaaaaaa gagaccgcct cgtttctttt tcttcgtcga aaaaggcaat    1260

aaaaattttt atcacgtttc tttttcttga aatttttttt tttagttttt ttctctttca    1320

gtgacctcca ttgatattta agttaataaa cggtcttcaa tttctcaagt ttcagtttca    1380

tttttcttgt tctattacaa ctttttttac ttcttgttca ttagaaagaa agcatagcaa    1440

tctaatctaa ggggcggtgt tgacaattaa tcatcggcat agtatatcgg catagtataa    1500

tacgacaagg tgaggaacta aaccatggcc aagttgacca gtgccgttcc ggtgctcacc    1560

gcgcgcgacg tcgccggagc ggtcgagttc tggaccgacc ggctcgggtt ctcccgggac    1620

ttcgtggagg acgacttcgc cggtgtggtc cgggacgacg tgaccctgtt catcagcgcg    1680

gtccaggacc aggtggtgcc ggacaacacc ctggcctggg tgtgggtgcg cggcctggac    1740

gagctgtacg ccgagtggtc ggaggtcgtg tccacgaact tccgggacgc ctccgggccg    1800

gccatgaccg agatcggcga gcagccgtgg gggcgggagt tcgccctgcg cgacccggcc 1860

ggcaactgcg tgcacttcgt ggccgaggag caggactgac acgtccgacg gcggcccacg 1920

ggtcccaggc ctcggagatc cgtccccctt ttcctttgtc gatatcatgt aattagttat 1980

gtcacgctta cattcacgcc ctccccccac atccgctcta accgaaaagg aaggagttag 2040

acaacctgaa gtctaggtcc ctatttattt ttttatagtt atgttagtat taagaacgtt 2100

atttatattt caaatttttc ttttttttct gtacagacgc gtgtacgcat gtaacattat 2160

actgaaaacc ttgcttgaga aggttttggg acgctcgaag gctttaattt gcaagctgga 2220

gaccaacatg tgagcaaaag gccagcaaaa ggccaggaac cgtaaaaagg ccgcgttgct 2280

ggcgtttttc cataggctcc gcccccctga cgagcatcac aaaaatcgac gctcaagtca 2340

gaggtggcga aacccgacag gactataaag ataccaggcg tttccccctg gaagctccct 2400

cgtgcgctct cctgttccga ccctgccgct taccggatac ctgtccgcct ttctcccttc 2460

gggaagcgtg gcgctttctc aatgctcacg ctgtaggtat ctcagttcgg tgtaggtcgt 2520

tcgctccaag ctgggctgtg tgcacgaacc ccccgttcag cccgaccgct gcgccttatc 2580

cggtaactat cgtcttgagt ccaacccggt aagacacgac ttatcgccac tggcagcagc 2640

cactggtaac aggattagca gagcgaggta tgtaggcggt gctacagagt tcttgaagtg 2700

gtggcctaac tacggctaca ctagaaggac agtatttggt atctgcgctc tgctgaagcc 2760

agttaccttc ggaaaaagag ttggtagctc ttgatccggc aaacaaacca ccgctggtag 2820

cggtggtttt tttgtttgca agcagcagat tacgcgcaga aaaaaaggat ctcaagaaga 2880

tcctttgatc ttttctacgg ggtctgacgc tcagtggaac gaaaactcac gttaagggat 2940

tttggtcatg agatcagatc taacatccaa agacgaaagg ttgaatgaaa cctttttgcc 3000

atccgacatc cacaggtcca ttctcacaca taagtgccaa acgcaacagg aggggataca 3060

ctagcagcag accgttgcaa acgcaggacc tccactcctc ttctcctcaa cacccacttt 3120

tgccatcgaa aaaccagccc agttattggg cttgattgga gctcgctcat tccaattcct 3180

tctattaggc tactaacacc atgactttat tagcctgtct atcctggccc ccctggcgag 3240

gttcatgttt gtttatttcc gaatgcaaca agctccgcat tacacccgaa catcactcca 3300

gatgagggct ttctgagtgt ggggtcaaat agtttcatgt tccccaaatg gcccaaaact 3360

gacagtttaa acgctgtctt ggaacctaat atgacaaaag cgtgatctca tccaagatga 3420

actaagtttg gttcgttgaa atgctaacgg ccagttggtc aaaaagaaac ttccaaaagt 3480

cggcataccg tttgtcttgt ttggtattga ttgacgaatg ctcaaaaata atctcattaa 3540

tgcttagcgc agtctctcta tcgcttctga accccggtgc acctgtgccg aaacgcaaat 3600

ggggaaacac ccgctttttg gatgattatg cattgtctcc acattgtatg cttccaagat 3660

tctggtggga atactgctga tagcctaacg ttcatgatca aaatttaact gttctaaccc 3720

ctacttgaca gcaatatata aacagaagga agctgccctg tcttaaacct ttttttttt 3778

<210> 23
<211> 3856
<212> DNA
<213> Artificial Sequence

<220>
<223> pPICZd-sp-N-(PSc)-mSes iLTP2

<400> 23
atcatcatta ttagcttact ttcataattg cgactggttc caattgacaa gcttttgatt 60

ttaacgactt ttaacgacaa cttgagaaga tcaaaaaaca actaattatt cgaaacgatg 120

agatttcctt caatttttac tgctgtttta ttcgcagcat cctccgcatt agctgctcca 180

gtcaacacta caacagaaga tgaaacggca caaattccgg ctgaagctgt catcggttac 240

tcagatttag aaggggattt cgatgttgct gttttgccat tttccaacag cacaaataac 300

gggttattgt ttataaatac tactattgcc agcattgctg ctaaagaaga aggggtatct 360

ttagagaaaa gagccatggc tcatcatcat catcatcatg gcggtggaag tggaggaggt 420

agtggaggcg ggtcacttga agtcttgttt caaggtccat ctgccatggc catatcatgc 480

ggcactgtgg ccaacgacct gtccccgtgc atcaactacg tgatgtatgg tggggcgggg 540

acgccaccgg taccatgctg caccgggata aagaccctct acaatcaggc aagcagcacc 600

gctgaccgcc aggctgtttg tgggtgcttg aaatctgttg ccggccaagc cagccctgcc 660

atcattggca cagccgccgc acttcctggc aaatgtggcg tctctattcc ttacgagatc 720

agcccctcca cggattgctc caaggttcat taactcgacc atcatcatca tcatcattga 780

gtttgtagcc ttagacatga ctgttcctca gttcaagttg ggcacttacg agaagaccgg 840

tcttgctaga ttctaatcaa gaggatgtca gaatgccatt tgcctgagag atgcaggctt 900

catttttgat acttttttat ttgtaaccta tatagtatag gatttttttt gtcattttgt 960

ttcttctcgt acgagcttgc tcctgatcag cctatctcgc agctgatgaa tatcttgtgg 1020

taggggtttg ggaaaatcat tcgagtttga tgtttttctt ggtatttccc actcctcttc 1080

agagtacaga agattaagtg agaccttcgt ttgtgcggat cccccacaca ccatagcttc 1140

aaaatgtttc tactcctttt ttactcttcc agattttctc ggactccgcg catcgccgta 1200

ccacttcaaa acacccaagc acagcatact aaatttcccc tctttcttcc tctagggtgt 1260

cgttaattac ccgtactaaa ggtttggaaa agaaaaaaga gaccgcctcg tttctttttc 1320

ttcgtcgaaa aaggcaataa aaatttttat cacgtttctt tttcttgaaa tttttttttt 1380

tagttttttt ctctttcagt gacctccatt gatatttaag ttaataaacg gtcttcaatt 1440

tctcaagttt cagtttcatt tttcttgttc tattacaact ttttttactt cttgttcatt 1500

agaaagaaag catagcaatc taatctaagg ggcggtgttg acaattaatc atcggcatag 1560

tatatcggca tagtataata cgacaaggtg aggaactaaa ccatggccaa gttgaccagt 1620

gccgttccgg tgctcaccgc gcgcgacgtc gccggagcgg tcgagttctg gaccgaccgg 1680

ctcgggttct cccgggactt cgtggaggac gacttcgccg gtgtggtccg ggacgacgtg 1740

accctgttca tcagcgcggt ccaggaccag gtggtgccgg acaacaccct ggcctgggtg 1800

tgggtgcgcg gcctggacga gctgtacgcc gagtggtcgg aggtcgtgtc cacgaacttc 1860

cgggacgcct ccgggccggc catgaccgag atcggcgagc agccgtgggg gcgggagttc 1920

gccctgcgcg acccggccgg caactgcgtg cacttcgtgg ccgaggagca ggactgacac 1980

gtccgacggc ggcccacggg tcccaggcct cggagatccg tccccctttt cctttgtcga 2040

tatcatgtaa ttagttatgt cacgcttaca ttcacgccct ccccccacat ccgctctaac 2100

cgaaaaggaa ggagttagac aacctgaagt ctaggtccct atttattttt ttatagttat 2160

gttagtatta agaacgttat ttatatttca aatttttctt ttttttctgt acagacgcgt 2220

gtacgcatgt aacattatac tgaaaacctt gcttgagaag gttttgggac gctcgaaggc 2280

tttaatttgc aagctggaga ccaacatgtg agcaaaaggc cagcaaaagg ccaggaaccg 2340

taaaaaggcc gcgttgctgg cgtttttcca taggctccgc ccccctgacg agcatcacaa 2400

aaatcgacgc tcaagtcaga ggtggcgaaa cccgacagga ctataaagat accaggcgtt 2460

tccccctgga agctccctcg tgcgctctcc tgttccgacc ctgccgctta ccggatacct 2520

gtccgccttt ctcccttcgg gaagcgtggc gctttctcaa tgctcacgct gtaggtatct 2580

cagttcggtg taggtcgttc gctccaagct gggctgtgtg cacgaacccc ccgttcagcc 2640

cgaccgctgc gccttatccg gtaactatcg tcttgagtcc aacccggtaa gacacgactt 2700

atcgccactg gcagcagcca ctggtaacag gattagcaga gcgaggtatg taggcggtgc 2760

tacagagttc ttgaagtggt ggcctaacta cggctacact agaaggacag tatttggtat 2820

ctgcgctctg ctgaagccag ttaccttcgg aaaaagagtt ggtagctctt gatccggcaa 2880

acaaaccacc gctggtagcg gtggtttttt tgtttgcaag cagcagatta cgcgcagaaa 2940

aaaaggatct caagaagatc ctttgatctt ttctacgggg tctgacgctc agtggaacga 3000

aaactcacgt taagggattt tggtcatgag atcagatcta acatccaaag acgaaaggtt 3060

gaatgaaacc tttttgccat ccgacatcca caggtccatt ctcacacata agtgccaaac 3120

gcaacaggag gggatacact agcagcagac cgttgcaaac gcaggacctc cactcctctt 3180

ctcctcaaca cccacttttg ccatcgaaaa accagcccag ttattgggct tgattggagc 3240

tcgctcattc caattccttc tattaggcta ctaacaccat gactttatta gcctgtctat 3300

cctggccccc ctggcgaggt tcatgtttgt ttatttccga atgcaacaag ctccgcatta 3360

cacccgaaca tcactccaga tgagggcttt ctgagtgtgg ggtcaaatag tttcatgttc 3420

cccaaatggc ccaaaactga cagtttaaac gctgtcttgg aacctaatat gacaaaagcg 3480

tgatctcatc caagatgaac taagtttggt tcgttgaaat gctaacggcc agttggtcaa 3540

aaagaaactt ccaaaagtcg gcataccgtt tgtcttgttt ggtattgatt gacgaatgct 3600

caaaaataat ctcattaatg cttagcgcag tctctctatc gcttctgaac cccggtgcac 3660

ctgtgccgaa acgcaaatgg ggaaacaccc gctttttgga tgattatgca ttgtctccac 3720

attgtatgct tccaagattc tggtgggaat actgctgata gcctaacgtt catgatcaaa 3780

atttaactgt tctaacccct acttgacagc aatatataaa cagaaggaag ctgccctgtc 3840

ttaaaccttt tttttt 3856

<210> 24
<211> 3847
<212> DNA
<213> Artificial Sequence

<220>
<223> pPICZd-sp-N-(PSc)-mSes iLTP3

<400> 24
atcatcatta ttagcttact ttcataattg cgactggttc caattgacaa gcttttgatt 60

ttaacgactt ttaacgacaa cttgagaaga tcaaaaaaca actaattatt cgaaacgatg 120

agatttcctt caatttttac tgctgtttta ttcgcagcat cctccgcatt agctgctcca 180

gtcaacacta caacagaaga tgaaacggca caaattccgg ctgaagctgt catcggttac 240

tcagatttag aaggggattt cgatgttgct gttttgccat tttccaacag cacaaataac 300

gggttattgt ttataaatac tactattgcc agcattgctg ctaaagaaga aggggtatct 360

ttagagaaaa gagccatggc tcatcatcat catcatcatg gcggtggaag tggaggaggt 420

agtggaggcg ggtcacttga agtcttgttt caaggtccat ctgccatggc gataggttgc 480

ggcactgtag tttcatatct gaattcatgc tttccatacg tgaccggaac tggtgcgttg 540

ggtggatgct gtaaagggat taaggatctc tatgcggcgg cccaaaccac agccgaccga 600

cagagcgtgt gcgactgcct gaaagatctt gctgcctcga ccactggcgt cgacctcggc 660

aaagctgctg gactccccag ccagtgtggt cttaacatcc cctacaagat cattccttca 720

actgattgct caaaggtgca atgactcgac catcatcatc atcatcattg agtttgtagc 780

cttagacatg actgttcctc agttcaagtt gggcacttac gagaagaccg gtcttgctag 840

attctaatca agaggatgtc agaatgccat ttgcctgaga gatgcaggct tcatttttga 900

tactttttta tttgtaacct atatagtata ggattttttt tgtcattttg tttcttctcg 960

tacgagcttg ctcctgatca gcctatctcg cagctgatga atatcttgtg gtaggggttt 1020

gggaaaatca ttcgagtttg atgtttttct tggtatttcc cactcctctt cagagtacag 1080

aagattaagt gagaccttcg tttgtgcgga tcccccacac accatagctt caaaatgttt 1140

ctactccttt tttactcttc cagattttct cggactccgc gcatcgccgt accacttcaa 1200

aacacccaag cacagcatac taaatttccc ctctttcttc ctctagggtg tcgttaatta 1260

cccgtactaa aggtttggaa aagaaaaaag agaccgcctc gtttcttttt cttcgtcgaa 1320

aaaggcaata aaaattttta tcacgtttct ttttcttgaa attttttttt ttagtttttt 1380

tctctttcag tgacctccat tgatatttaa gttaataaac ggtcttcaat ttctcaagtt 1440

tcagtttcat ttttcttgtt ctattacaac tttttttact tcttgttcat tagaaagaaa 1500

gcatagcaat ctaatctaag gggcggtgtt gacaattaat catcggcata gtatatcggc 1560

atagtataat acgacaaggt gaggaactaa accatggcca agttgaccag tgccgttccg 1620

gtgctcaccg cgcgcgacgt cgccggagcg gtcgagttct ggaccgaccg gctcgggttc 1680

tcccgggact tcgtggagga cgacttcgcc ggtgtggtcc gggacgacgt gaccctgttc 1740

atcagcgcgg tccaggacca ggtggtgccg gacaacaccc tggcctgggt gtgggtgcgc 1800

ggcctggacg agctgtacgc cgagtggtcg gaggtcgtgt ccacgaactt ccgggacgcc 1860

tccgggccgg ccatgaccga gatcggcgag cagccgtggg ggcgggagtt cgccctgcgc 1920

gacccggccg gcaactgcgt gcacttcgtg gccgaggagc aggactgaca cgtccgacgg 1980

cggcccacgg gtcccaggcc tcggagatcc gtcccccttt tcctttgtcg atatcatgta 2040

attagttatg tcacgcttac attcacgccc tccccccaca tccgctctaa ccgaaaagga 2100

aggagttaga caacctgaag tctaggtccc tatttatttt tttatagtta tgttagtatt 2160

aagaacgtta tttatatttc aaatttttct tttttttctg tacagacgcg tgtacgcatg 2220

taacattata ctgaaaacct tgcttgagaa ggttttggga cgctcgaagg ctttaatttg 2280

caagctggag accaacatgt gagcaaaagg ccagcaaaag gccaggaacc gtaaaaaggc 2340

cgcgttgctg gcgtttttcc ataggctccg cccccctgac gagcatcaca aaaatcgacg 2400

ctcaagtcag aggtggcgaa acccgacagg actataaaga taccaggcgt ttccccctgg 2460

aagctccctc gtgcgctctc ctgttccgac cctgccgctt accggatacc tgtccgcctt 2520

tctcccttcg ggaagcgtgg cgctttctca atgctcacgc tgtaggtatc tcagttcggt 2580

gtaggtcgtt cgctccaagc tgggctgtgt gcacgaaccc cccgttcagc ccgaccgctg 2640

cgccttatcc ggtaactatc gtcttgagtc caacccggta agacacgact tatcgccact 2700

ggcagcagcc actggtaaca ggattagcag agcgaggtat gtaggcggtg ctacagagtt 2760

cttgaagtgg tggcctaact acggctacac tagaaggaca gtatttggta tctgcgctct 2820

gctgaagcca gttaccttcg gaaaaagagt tggtagctct tgatccggca aacaaaccac 2880

cgctggtagc ggtggttttt ttgtttgcaa gcagcagatt acgcgcagaa aaaaaggatc 2940

tcaagaagat cctttgatct tttctacggg gtctgacgct cagtggaacg aaaactcacg 3000

ttaagggatt ttggtcatga gatcagatct aacatccaaa gacgaaaggt tgaatgaaac 3060

ctttttgcca tccgacatcc acaggtccat tctcacacat aagtgccaaa cgcaacagga 3120

ggggatacac tagcagcaga ccgttgcaaa cgcaggacct ccactcctct tctcctcaac 3180

acccactttt gccatcgaaa aaccagccca gttattgggc ttgattggag ctcgctcatt 3240

ccaattcctt ctattaggct actaacacca tgactttatt agcctgtcta tcctggcccc 3300

cctggcgagg ttcatgtttg tttatttccg aatgcaacaa gctccgcatt acacccgaac 3360

atcactccag atgagggctt tctgagtgtg gggtcaaata gtttcatgtt ccccaaatgg 3420

cccaaaactg acagtttaaa cgctgtcttg gaacctaata tgacaaaagc gtgatctcat 3480

ccaagatgaa ctaagtttgg ttcgttgaaa tgctaacggc cagttggtca aaaagaaact 3540

tccaaaagtc ggcataccgt ttgtcttgtt tggtattgat tgacgaatgc tcaaaaataa 3600

tctcattaat gcttagcgca gtctctctat cgcttctgaa ccccggtgca cctgtgccga 3660

aacgcaaatg gggaaacacc cgctttttgg atgattatgc attgtctcca cattgtatgc 3720

ttccaagatt ctggtgggaa tactgctgat agcctaacgt tcatgatcaa aatttaactg 3780

ttctaacccc tacttgacag caatatataa acagaaggaa gctgccctgt cttaaacctt 3840

ttttttt 3847

<210> 25
<211> 3847
<212> DNA
<213> Artificial Sequence

<220>
<223> pPICZd-sp-N-(PSc)-mSes iLTP4

<400> 25
atcatcatta ttagcttact ttcataattg cgactggttc caattgacaa gcttttgatt 60

ttaacgactt ttaacgacaa cttgagaaga tcaaaaaaca actaattatt cgaaacgatg 120

agatttcctt caatttttac tgctgtttta ttcgcagcat cctccgcatt agctgctcca 180

gtcaacacta caacagaaga tgaaacggca caaattccgg ctgaagctgt catcggttac 240

tcagatttag aaggggattt cgatgttgct gttttgccat tttccaacag cacaaataac 300

gggttattgt ttataaatac tactattgcc agcattgctg ctaaagaaga aggggtatct 360

ttagagaaaa gagccatggc tcatcatcat catcatcatg gcggtggaag tggaggaggt 420

agtggaggcg ggtcacttga agtcttgttt caaggtccat ctgccatggc aataggctgc 480

ggcacggtgg tttcatacct gaatccatgc ctgccatatg taaccaacaa aggccctcta 540

ggcagttgtt gcggtggagt taagggtctg tatggagcag cccagactgc acaagatcgt 600

caaagcgtgt gcagctgctt gaaatctctc gctggctcat acaaagatgt cgacctgaac 660

aaggccgctg gactccctgg acaatgtggc gtcaacattc cctacaagat tagcccttcc 720

actgattgct caaaggtgaa ttgactcgac catcatcatc atcatcattg agtttgtagc 780

cttagacatg actgttcctc agttcaagtt gggcacttac gagaagaccg gtcttgctag 840

attctaatca agaggatgtc agaatgccat ttgcctgaga gatgcaggct tcatttttga 900

tactttttta tttgtaacct atatagtata ggattttttt tgtcattttg tttcttctcg 960

tacgagcttg ctcctgatca gcctatctcg cagctgatga atatcttgtg gtaggggttt 1020

gggaaaatca ttcgagtttg atgtttttct tggtatttcc cactcctctt cagagtacag 1080

aagattaagt gagaccttcg tttgtgcgga tcccccacac accatagctt caaaatgttt 1140

ctactccttt tttactcttc cagattttct cggactccgc gcatcgccgt accacttcaa 1200

aacacccaag cacagcatac taaatttccc ctctttcttc ctctagggtg tcgttaatta 1260

cccgtactaa aggtttggaa aagaaaaaag agaccgcctc gtttcttttt cttcgtcgaa 1320

aaaggcaata aaaattttta tcacgtttct ttttcttgaa attttttttt ttagtttttt 1380

tctctttcag tgacctccat tgatatttaa gttaataaac ggtcttcaat ttctcaagtt 1440

tcagtttcat ttttcttgtt ctattacaac tttttttact tcttgttcat tagaaagaaa 1500

gcatagcaat ctaatctaag gggcggtgtt gacaattaat catcggcata gtatatcggc 1560

atagtataat acgacaaggt gaggaactaa accatggcca agttgaccag tgccgttccg 1620

gtgctcaccg cgcgcgacgt cgccggagcg gtcgagttct ggaccgaccg gctcgggttc 1680

tcccgggact tcgtggagga cgacttcgcc ggtgtggtcc gggacgacgt gaccctgttc 1740

atcagcgcgg tccaggacca ggtggtgccg gacaacaccc tggcctgggt gtgggtgcgc 1800

ggcctggacg agctgtacgc cgagtggtcg gaggtcgtgt ccacgaactt ccgggacgcc 1860

tccgggccgg ccatgaccga gatcggcgag cagccgtggg ggcgggagtt cgccctgcgc 1920

gacccggccg gcaactgcgt gcacttcgtg gccgaggagc aggactgaca cgtccgacgg 1980

cggcccacgg gtcccaggcc tcggagatcc gtcccccttt tcctttgtcg atatcatgta 2040

attagttatg tcacgcttac attcacgccc tccccccaca tccgctctaa ccgaaaagga 2100

aggagttaga caacctgaag tctaggtccc tatttatttt tttatagtta tgttagtatt 2160

aagaacgtta tttatatttc aaatttttct tttttttctg tacagacgcg tgtacgcatg 2220

taacattata ctgaaaacct tgcttgagaa ggttttggga cgctcgaagg ctttaatttg 2280

caagctggag accaacatgt gagcaaaagg ccagcaaaag gccaggaacc gtaaaaaggc 2340

cgcgttgctg gcgtttttcc ataggctccg cccccctgac gagcatcaca aaaatcgacg 2400

ctcaagtcag aggtggcgaa acccgacagg actataaaga taccaggcgt ttccccctgg 2460

aagctccctc gtgcgctctc ctgttccgac cctgccgctt accggatacc tgtccgcctt 2520

tctcccttcg ggaagcgtgg cgctttctca atgctcacgc tgtaggtatc tcagttcggt 2580

gtaggtcgtt cgctccaagc tgggctgtgt gcacgaaccc cccgttcagc ccgaccgctg 2640

cgccttatcc ggtaactatc gtcttgagtc caacccggta agacacgact tatcgccact 2700

ggcagcagcc actggtaaca ggattagcag agcgaggtat gtaggcggtg ctacagagtt 2760

cttgaagtgg tggcctaact acggctacac tagaaggaca gtatttggta tctgcgctct 2820

gctgaagcca gttaccttcg gaaaaagagt tggtagctct tgatccggca aacaaaccac 2880

cgctggtagc ggtggttttt ttgtttgcaa gcagcagatt acgcgcagaa aaaaaggatc 2940

tcaagaagat cctttgatct tttctacggg gtctgacgct cagtggaacg aaaactcacg 3000

ttaagggatt ttggtcatga gatcagatct aacatccaaa gacgaaaggt tgaatgaaac 3060

ctttttgcca tccgacatcc acaggtccat tctcacacat aagtgccaaa cgcaacagga 3120

ggggatacac tagcagcaga ccgttgcaaa cgcaggacct ccactcctct tctcctcaac 3180

acccactttt gccatcgaaa aaccagccca gttattgggc ttgattggag ctcgctcatt 3240

ccaattcctt ctattaggct actaacacca tgactttatt agcctgtcta tcctggcccc 3300

cctggcgagg ttcatgtttg tttatttccg aatgcaacaa gctccgcatt acacccgaac 3360

atcactccag atgagggctt tctgagtgtg gggtcaaata gtttcatgtt ccccaaatgg 3420

cccaaaactg acagtttaaa cgctgtcttg gaacctaata tgacaaaagc gtgatctcat 3480

ccaagatgaa ctaagtttgg ttcgttgaaa tgctaacggc cagttggtca aaaagaaact 3540

tccaaaagtc ggcataccgt ttgtcttgtt tggtattgat tgacgaatgc tcaaaaataa 3600

tctcattaat gcttagcgca gtctctctat cgcttctgaa ccccggtgca cctgtgccga 3660

aacgcaaatg gggaaacacc cgctttttgg atgattatgc attgtctcca cattgtatgc 3720

ttccaagatt ctggtgggaa tactgctgat agcctaacgt tcatgatcaa aatttaactg 3780

ttctaacccc tacttgacag caatatataa acagaaggaa gctgccctgt cttaaacctt 3840

ttttttt 3847

<210> 26
<211> 3847
<212> DNA
<213> Artificial Sequence

<220>
<223> pPICZd-sp-N-(PSc)-mSes iLTP5

<400> 26
atcatcatta ttagcttact ttcataattg cgactggttc caattgacaa gcttttgatt 60

ttaacgactt ttaacgacaa cttgagaaga tcaaaaaaca actaattatt cgaaacgatg 120

agatttcctt caatttttac tgctgtttta ttcgcagcat cctccgcatt agctgctcca 180

gtcaacacta caacagaaga tgaaacggca caaattccgg ctgaagctgt catcggttac 240

tcagatttag aaggggattt cgatgttgct gttttgccat tttccaacag cacaaataac 300

gggttattgt ttataaatac tactattgcc agcattgctg ctaaagaaga aggggtatct 360

ttagagaaaa gagccatggc tcatcatcat catcatcatg gcggtggaag tggaggaggt 420

agtggaggcg ggtcacttga agtcttgttt caaggtccat ctgccatggc attaggctgc 480

ggcagcgtga tatcttatct gggttcatgc cgcccctatg tgaccgacaa aggtccttta 540

ggtggttgct gcagtggggt gaagggactc tataaggctg caaaaaccac cgcggatcgt 600

caggccactt gcagctgcct caaaactctt gccagtacct acaaaggtgt caatcttagc 660

aaggcagccg gacttcctca gcaatgtggt gttaacattc cctataaaat cagcccttcc 720

accgattgct caaaggtgac gtgactcgac catcatcatc atcatcattg agtttgtagc 780

cttagacatg actgttcctc agttcaagtt gggcacttac gagaagaccg gtcttgctag 840

attctaatca agaggatgtc agaatgccat ttgcctgaga gatgcaggct tcatttttga 900

tactttttta tttgtaacct atatagtata ggattttttt tgtcattttg tttcttctcg 960

tacgagcttg ctcctgatca gcctatctcg cagctgatga atatcttgtg gtaggggttt 1020

gggaaaatca ttcgagtttg atgtttttct tggtatttcc cactcctctt cagagtacag 1080

aagattaagt gagaccttcg tttgtgcgga tcccccacac accatagctt caaaatgttt 1140

ctactccttt tttactcttc cagattttct cggactccgc gcatcgccgt accacttcaa 1200

aacacccaag cacagcatac taaatttccc ctctttcttc ctctagggtg tcgttaatta 1260

cccgtactaa aggtttggaa aagaaaaaag agaccgcctc gtttcttttt cttcgtcgaa 1320

aaaggcaata aaaattttta tcacgtttct ttttcttgaa aatttttttt ttagtttttt 1380

tctctttcag tgacctccat tgatatttaa gttaataaac ggtcttcaat ttctcaagtt 1440

tcagtttcat ttttcttgtt ctattacaac tttttttact tcttgttcat tagaaagaaa 1500

gcatagcaat ctaatctaag gggcggtgtt gacaattaat catcggcata gtatatcggc 1560

atagtataat acgacaaggt gaggaactaa accatggcca agttgaccag tgccgttccg 1620

gtgctcaccg cgcgcgacgt cgccggagcg gtcgagttct ggaccgaccg gctcgggttc 1680

tcccgggact tcgtggagga cgacttcgcc ggtgtggtcc gggacgacgt gaccctgttc 1740

atcagcgcgg tccaggacca ggtggtgccg gacaacaccc tggcctgggt gtgggtgcgc 1800

ggcctggacg agctgtacgc cgagtggtcg gaggtcgtgt ccacgaactt ccgggacgcc 1860

tccgggccgg ccatgaccga gatcggcgag cagccgtggg ggcgggagtt cgccctgcgc 1920

gacccggccg gcaactgcgt gcacttcgtg gccgaggagc aggactgaca cgtccgacgg 1980

cggcccacgg gtcccaggcc tcggagatcc gtcccccttt tcctttgtcg atatcatgta 2040

attagttatg tcacgcttac attcacgccc tccccccaca tccgctctaa ccgaaaagga 2100

aggagttaga caacctgaag tctaggtccc tatttatttt tttatagtta tgttagtatt 2160

aagaacgtta tttatatttc aaatttttct tttttttctg tacagacgcg tgtacgcatg 2220

taacattata ctgaaaacct tgcttgagaa ggttttggga cgctcgaagg ctttaatttg 2280

caagctggag accaacatgt gagcaaaagg ccagcaaaag gccaggaacc gtaaaaaggc 2340

cgcgttgctg gcgtttttcc ataggctccg cccccctgac gagcatcaca aaaatcgacg 2400

ctcaagtcag aggtggcgaa acccgacagg actataaaga taccaggcgt ttccccctgg 2460

aagctccctc gtgcgctctc ctgttccgac cctgccgctt accggatacc tgtccgcctt 2520

tctcccttcg ggaagcgtgg cgctttctca atgctcacgc tgtaggtatc tcagttcggt 2580

gtaggtcgtt cgctccaagc tgggctgtgt gcacgaaccc cccgttcagc ccgaccgctg 2640

cgccttatcc ggtaactatc gtcttgagtc caacccggta agacacgact tatcgccact 2700

ggcagcagcc actggtaaca ggattagcag agcgaggtat gtaggcggtg ctacagagtt 2760

cttgaagtgg tggcctaact acggctacac tagaaggaca gtatttggta tctgcgctct 2820

gctgaagcca gttaccttcg gaaaaagagt tggtagctct tgatccggca aacaaaccac 2880

cgctggtagc ggtggttttt ttgtttgcaa gcagcagatt acgcgcagaa aaaaaggatc 2940

tcaagaagat cctttgatct tttctacggg gtctgacgct cagtggaacg aaaactcacg 3000

ttaagggatt ttggtcatga gatcagatct aacatccaaa gacgaaaggt tgaatgaaac 3060

ctttttgcca tccgacatcc acaggtccat tctcacacat aagtgccaaa cgcaacagga 3120

ggggatacac tagcagcaga ccgttgcaaa cgcaggacct ccactcctct tctcctcaac 3180

acccactttt gccatcgaaa aaccagccca gttattgggc ttgattggag ctcgctcatt 3240

ccaattcctt ctattaggct actaacacca tgactttatt agcctgtcta tcctggcccc 3300

cctggcgagg ttcatgtttg tttatttccg aatgcaacaa gctccgcatt acacccgaac 3360

atcactccag atgagggctt tctgagtgtg gggtcaaata gtttcatgtt ccccaaatgg 3420

cccaaaactg acagtttaaa cgctgtcttg gaacctaata tgacaaaagc gtgatctcat 3480

ccaagatgaa ctaagtttgg ttcgttgaaa tgctaacggc cagttggtca aaaagaaact 3540

tccaaaagtc ggcataccgt ttgtcttgtt tggtattgat tgacgaatgc tcaaaaataa 3600

tctcattaat gcttagcgca gtctctctat cgcttctgaa ccccggtgca cctgtgccga 3660

aacgcaaatg gggaaacacc cgctttttgg atgattatgc attgtctcca cattgtatgc 3720

ttccaagatt ctggtgggaa tactgctgat agcctaacgt tcatgatcaa aatttaactg 3780

ttctaacccc tacttgacag caatatataa acagaaggaa gctgccctgt cttaaacctt 3840

ttttttt 3847

<210> 27
<211> 17
<212> PRT

<213>  Artificial Sequence

<220>
<223>  epitope of LTP1

<400>  27

Ser Gly Ser Gln Pro Pro Ser Ala Glu Cys Cys Ser Lys Leu Lys Glu
1               5                   10                  15

Gln

<210>  28
<211>  11
<212>  PRT
<213>  Artificial Sequence

<220>
<223>  epitope of LTP1

<400>  28

Ala Ala Thr Cys Gly Val Ala Phe Pro Thr Cys
1               5                   10

<210>  29
<211>  20
<212>  PRT
<213>  Artificial Sequence

<220>
<223>  epitope of LTP2

<400>  29

Val Met Tyr Gly Gly Ala Gly Thr Pro Pro Val Pro Cys Cys Thr Gly
1               5                   10                  15

Ile Lys Thr Leu
            20

<210>  30
<211>  7
<212>  PRT
<213>  Artificial Sequence

<220>
<223>  epitope of LTP2

<400>  30

Ser Ser Thr Ala Asp Arg Gln
1               5

<210>  31
<211>  8

<212>  PRT
<213>  Artificial Sequence

<220>
<223>  epitope of LTP2

<400>  31

Pro Gly Lys Cys Gly Val Ser Ile
1               5


<210>  32
<211>  17
<212>  PRT
<213>  Artificial Sequence

<220>
<223>  epitope of LTP3

<400>  32

Val Thr Asp Lys Gly Pro Leu Gly Gly Cys Cys Ser Gly Val Lys Gly
1               5                   10                  15


Leu




<210>  33
<211>  7
<212>  PRT
<213>  Artificial Sequence

<220>
<223>  epitope of LTP3

<400>  33

Lys Thr Thr Ala Asp Arg Gln
1               5


<210>  34
<211>  8
<212>  PRT
<213>  Artificial Sequence

<220>
<223>  epitope of LTP3

<400>  34

Pro Gln Gln Cys Gly Val Asn Ile
1               5


<210>  35
<211>  17
<212>  PRT
<213>  Artificial Sequence

<220>
<223>  epitope of LTP4

<400>  35

Val Thr Gly Thr Gly Ala Leu Gly Gly Cys Cys Lys Gly Ile Lys Asp
1               5                   10                  15

Leu

<210>  36
<211>  7
<212>  PRT
<213>  Artificial Sequence

<220>
<223>  epitope of LTP4

<400>  36

Gln Thr Thr Ala Asp Arg Gln
1               5

<210>  37
<211>  8
<212>  PRT
<213>  Artificial Sequence

<220>
<223>  epitope of LTP4

<400>  37

Pro Ser Gln Cys Gly Leu Asn Ile
1               5

<210>  38
<211>  17
<212>  PRT
<213>  Artificial Sequence

<220>
<223>  epitope of LTP5

<400>  38

Val Thr Asn Lys Gly Pro Leu Gly Ser Cys Cys Gly Gly Val Lys Gly
1               5                   10                  15

Leu

<210>  39
<211>  7
<212>  PRT
<213>  Artificial Sequence

<220>
<223>  epitope of LTP5

<400>  39

Gln Thr Ala Gln Asp Arg Gln
1               5


<210>  40
<211>  8
<212>  PRT
<213>  Artificial Sequence

<220>
<223>  epitope of LTP5

<400>  40

Pro Gly Gln Cys Gly Val Asn Ile
1               5


<210>  41
<211>  74
<212>  PRT
<213>  Artificial Sequence

<220>
<223>  fragment of LTP2

<400>  41

Cys Gly Thr Val Ala Asn Asp Leu Ser Pro Cys Ile Asn Tyr Val Met
1               5                   10                  15


Tyr Gly Gly Ala Gly Thr Pro Pro Val Pro Cys Cys Thr Gly Ile Lys
            20                  25                  30


Thr Leu Tyr Asn Gln Ala Ser Ser Thr Ala Asp Arg Gln Ala Val Cys
        35                  40                  45


Gly Cys Leu Lys Ser Val Ala Gly Gln Ala Ser Pro Ala Ile Ile Gly
    50                  55                  60


Thr Ala Ala Ala Leu Pro Gly Lys Cys Gly
65                  70


<210>  42
<211>  71
<212>  PRT
<213>  Artificial Sequence

<220>
<223>  fragment of LTP3

<400>  42

Cys Gly Thr Val Val Ser Tyr Leu Asn Ser Cys Phe Pro Tyr Val Thr
1               5                   10                  15

Gly Thr Gly Ala Leu Gly Gly Cys Cys Lys Gly Ile Lys Asp Leu Tyr
            20                  25                  30

Ala Ala Ala Gln Thr Thr Ala Asp Arg Gln Ser Val Cys Asp Cys Leu
        35                  40                  45

Lys Asp Leu Ala Ala Ser Thr Thr Gly Val Asp Leu Gly Lys Ala Ala
    50                  55                  60

Gly Leu Pro Ser Gln Cys Gly
65                  70

<210>  43
<211>  71
<212>  PRT
<213>  Artificial Sequence

<220>
<223>  fragment of LTP4

<400>  43

Cys Gly Thr Val Val Ser Tyr Leu Asn Pro Cys Leu Pro Tyr Val Thr
1               5                   10                  15

Asn Lys Gly Pro Leu Gly Ser Cys Cys Gly Gly Val Lys Gly Leu Tyr
            20                  25                  30

Gly Ala Ala Gln Thr Ala Gln Asp Arg Gln Ser Val Cys Ser Cys Leu
        35                  40                  45

Lys Ser Leu Ala Gly Ser Tyr Lys Asp Val Asp Leu Asn Lys Ala Ala
    50                  55                  60

Gly Leu Pro Gly Gln Cys Gly
65                  70

<210>  44
<211>  71
<212>  PRT
<213>  Artificial Sequence

<220>
<223>  fragment of LTP5

<400>  44

Cys Gly Ser Val Ile Ser Tyr Leu Gly Ser Cys Arg Pro Tyr Val Thr
1               5                   10                  15

Asp Lys Gly Pro Leu Gly Gly Cys Cys Ser Gly Val Lys Gly Leu Tyr
            20                  25                  30

Lys Ala Ala Lys Thr Thr Ala Asp Arg Gln Ala Thr Cys Ser Cys Leu
        35                  40                  45

Lys Thr Leu Ala Ser Thr Tyr Lys Gly Val Asn Leu Ser Lys Ala Ala
    50                  55                  60

Gly Leu Pro Gln Gln Cys Gly
65                  70

<210>  45
<211>  91
<212>  PRT
<213>  Artificial Sequence

<220>
<223>  MutB

<400>  45

Ala Ile Gly Cys Gly Thr Val Val Ser Tyr Leu Asn Ser Cys Leu Pro
1               5                   10                  15

Tyr Val Thr Asn Lys Gly Pro Leu Gly Gly Cys Cys Gly Gly Val Lys
            20                  25                  30

Gly Leu Tyr Gly Ala Ala Gln Thr Thr Ala Asp Arg Gln Ser Val Cys
        35                  40                  45

Ser Cys Leu Lys Ser Leu Ala Ser Ser Tyr Lys Gly Val Asp Leu Ser
    50                  55                  60

Lys Ala Ala Gly Leu Pro Gly Gln Cys Gly Val Asn Ile Pro Tyr Lys
65                  70                  75                  80

Ile Ser Pro Ser Thr Asp Cys Ser Lys Val Asn
                85                  90

<210>  46
<211>  91
<212>  PRT
<213>  Artificial Sequence

<220>
<223>  MutD

<400>  46

Ala Ile Gly Cys Gly Thr Val Val Ser Tyr Leu Asn Ser Cys Arg Pro

1                   5                   10                  15

Tyr Val Thr Asp Lys Gly Pro Leu Gly Gly Cys Cys Ser Gly Ile Lys
            20                  25                  30

Gly Leu Tyr Lys Ala Ala Gln Thr Thr Ala Asp Arg Gln Ser Val Cys
        35                  40                  45

Ser Cys Leu Lys Thr Leu Ala Ala Ser Tyr Lys Gly Val Asp Leu Gly
    50                  55                  60

Lys Ala Ala Gly Leu Pro Gln Gln Cys Gly Val Asn Ile Pro Tyr Lys
65                  70                  75                  80

Ile Ser Pro Ser Thr Asp Cys Ser Lys Val Thr
                85                  90

<210>  47
<211>  94
<212>  PRT
<213>  Artificial Sequence

<220>
<223>  MutM

<400>  47

Ala Leu Ser Cys Gly Ser Val Ala Ser Asp Leu Ser Pro Cys Ile Asn
1                   5                   10                  15

Tyr Val Met Tyr Gly Gly Ala Gly Thr Pro Pro Val Pro Cys Cys Ser
            20                  25                  30

Gly Val Lys Gly Leu Tyr Lys Gln Ala Lys Ser Thr Ala Asp Arg Gln
        35                  40                  45

Ala Thr Cys Ser Cys Leu Lys Thr Leu Ala Ser Thr Ala Ser Pro Ala
    50                  55                  60

Ile Leu Ser Thr Ala Ala Ala Leu Pro Gly Gln Cys Gly Val Asn Ile
65                  70                  75                  80

Pro Tyr Lys Ile Ser Pro Ser Thr Asp Cys Ser Lys Val Thr
                85                  90

<210>  48
<211>  94
<212>  PRT
<213>  Artificial Sequence

<220>

<223>  MutN

<400>  48

Ala Leu Ser Cys Gly Ser Val Ala Asn Asp Leu Ser Pro Cys Ile Asn
1               5                   10                  15

Tyr Val Met Tyr Gly Gly Ala Gly Thr Pro Pro Val Pro Cys Cys Ser
            20                  25                  30

Gly Val Lys Gly Leu Tyr Lys Gln Ala Ser Ser Thr Ala Asp Arg Gln
        35                  40                  45

Ala Val Cys Gly Cys Leu Lys Thr Leu Ala Gly Thr Ala Ser Pro Ala
    50                  55                  60

Ile Leu Gly Thr Ala Ala Ala Leu Pro Gly Gln Cys Gly Val Ser Ile
65                  70                  75                  80

Pro Tyr Glu Ile Ser Pro Ser Thr Asp Cys Ser Lys Val Thr
                85                  90

<210>  49
<211>  208
<212>  PRT
<213>  Artificial Sequence

<220>
<223>  alpha-factor-sp-H6-linker-PSc-MutB

<400>  49

Met Arg Phe Pro Ser Ile Phe Thr Ala Val Leu Phe Ala Ala Ser Ser
1               5                   10                  15

Ala Leu Ala Ala Pro Val Asn Thr Thr Thr Glu Asp Glu Thr Ala Gln
            20                  25                  30

Ile Pro Ala Glu Ala Val Ile Gly Tyr Ser Asp Leu Glu Gly Asp Phe
        35                  40                  45

Asp Val Ala Val Leu Pro Phe Ser Asn Ser Thr Asn Asn Gly Leu Leu
    50                  55                  60

Phe Ile Asn Thr Thr Ile Ala Ser Ile Ala Ala Lys Glu Glu Gly Val
65                  70                  75                  80

Ser Leu Glu Lys Arg Ala Met Ala His His His His His His Gly Gly
                85                  90                  95

Gly Ser Gly Gly Gly Ser Gly Gly Gly Ser Leu Glu Val Leu Phe Gln

100                 105                 110

Gly Pro Ser Ala Met Ala Ile Gly Cys Gly Thr Val Val Ser Tyr Leu
        115                 120                 125

Asn Ser Cys Leu Pro Tyr Val Thr Asn Lys Gly Pro Leu Gly Gly Cys
    130                 135                 140

Cys Gly Gly Val Lys Gly Leu Tyr Gly Ala Ala Gln Thr Thr Ala Asp
145                 150                 155                 160

Arg Gln Ser Val Cys Ser Cys Leu Lys Ser Leu Ala Ser Ser Tyr Lys
                165                 170                 175

Gly Val Asp Leu Ser Lys Ala Ala Gly Leu Pro Gly Gln Cys Gly Val
            180                 185                 190

Asn Ile Pro Tyr Lys Ile Ser Pro Ser Thr Asp Cys Ser Lys Val Asn
        195                 200                 205

<210>  50
<211>  208
<212>  PRT
<213>  Artificial Sequence

<220>
<223>  alpha-factor-sp-H6-linker-PSc-MutD

<400>  50

Met Arg Phe Pro Ser Ile Phe Thr Ala Val Leu Phe Ala Ala Ser Ser
1               5                   10                  15

Ala Leu Ala Ala Pro Val Asn Thr Thr Thr Glu Asp Glu Thr Ala Gln
            20                  25                  30

Ile Pro Ala Glu Ala Val Ile Gly Tyr Ser Asp Leu Glu Gly Asp Phe
        35                  40                  45

Asp Val Ala Val Leu Pro Phe Ser Asn Ser Thr Asn Asn Gly Leu Leu
    50                  55                  60

Phe Ile Asn Thr Thr Ile Ala Ser Ile Ala Ala Lys Glu Glu Gly Val
65                  70                  75                  80

Ser Leu Glu Lys Arg Ala Met Ala His His His His His His Gly Gly
                85                  90                  95

Gly Ser Gly Gly Gly Ser Gly Gly Gly Ser Leu Glu Val Leu Phe Gln
            100                 105                 110

Gly Pro Ser Ala Met Ala Ile Gly Cys Gly Thr Val Val Ser Tyr Leu
        115                 120                 125

Asn Ser Cys Arg Pro Tyr Val Thr Asp Lys Gly Pro Leu Gly Gly Cys
    130                 135                 140

Cys Ser Gly Ile Lys Gly Leu Tyr Lys Ala Ala Gln Thr Thr Ala Asp
145                 150                 155                 160

Arg Gln Ser Val Cys Ser Cys Leu Lys Thr Leu Ala Ala Ser Tyr Lys
                165                 170                 175

Gly Val Asp Leu Gly Lys Ala Ala Gly Leu Pro Gln Gln Cys Gly Val
            180                 185                 190

Asn Ile Pro Tyr Lys Ile Ser Pro Ser Thr Asp Cys Ser Lys Val Thr
        195                 200                 205

<210>  51
<211>  211
<212>  PRT
<213>  Artificial Sequence

<220>
<223>  alpha-factor-sp-H6-linker-PSc-MutM

<400>  51

Met Arg Phe Pro Ser Ile Phe Thr Ala Val Leu Phe Ala Ala Ser Ser
1               5                   10                  15

Ala Leu Ala Ala Pro Val Asn Thr Thr Thr Glu Asp Glu Thr Ala Gln
            20                  25                  30

Ile Pro Ala Glu Ala Val Ile Gly Tyr Ser Asp Leu Glu Gly Asp Phe
        35                  40                  45

Asp Val Ala Val Leu Pro Phe Ser Asn Ser Thr Asn Asn Gly Leu Leu
    50                  55                  60

Phe Ile Asn Thr Thr Ile Ala Ser Ile Ala Ala Lys Glu Glu Gly Val
65                  70                  75                  80

Ser Leu Glu Lys Arg Ala Met Ala His His His His His His Gly Gly
                85                  90                  95

Gly Ser Gly Gly Gly Ser Gly Gly Gly Ser Leu Glu Val Leu Phe Gln
            100                 105                 110

Gly Pro Ser Ala Met Ala Leu Ser Cys Gly Ser Val Ala Ser Asp Leu
        115                 120                 125

Ser Pro Cys Ile Asn Tyr Val Met Tyr Gly Gly Ala Gly Thr Pro Pro
    130                 135                 140

Val Pro Cys Cys Ser Gly Val Lys Gly Leu Tyr Lys Gln Ala Lys Ser
145                 150                 155                 160

Thr Ala Asp Arg Gln Ala Thr Cys Ser Cys Leu Lys Thr Leu Ala Ser
                165                 170                 175

Thr Ala Ser Pro Ala Ile Leu Ser Thr Ala Ala Ala Leu Pro Gly Gln
            180                 185                 190

Cys Gly Val Asn Ile Pro Tyr Lys Ile Ser Pro Ser Thr Asp Cys Ser
        195                 200                 205

Lys Val Thr
    210

<210> 52
<211> 211
<212> PRT
<213> Artificial Sequence

<220>
<223> alpha-factor-sp-H6-linker-PSc-MutN

<400> 52

Met Arg Phe Pro Ser Ile Phe Thr Ala Val Leu Phe Ala Ala Ser Ser
1               5                   10                  15

Ala Leu Ala Ala Pro Val Asn Thr Thr Thr Glu Asp Glu Thr Ala Gln
            20                  25                  30

Ile Pro Ala Glu Ala Val Ile Gly Tyr Ser Asp Leu Glu Gly Asp Phe
        35                  40                  45

Asp Val Ala Val Leu Pro Phe Ser Asn Ser Thr Asn Asn Gly Leu Leu
    50                  55                  60

Phe Ile Asn Thr Thr Ile Ala Ser Ile Ala Ala Lys Glu Glu Gly Val
65                  70                  75                  80

Ser Leu Glu Lys Arg Ala Met Ala His His His His His His Gly Gly
                85                  90                  95

Gly Ser Gly Gly Gly Ser Gly Gly Gly Ser Leu Glu Val Leu Phe Gln
            100                 105                 110

Gly Pro Ser Ala Met Ala Leu Ser Cys Gly Ser Val Ala Asn Asp Leu
        115                 120                 125

Ser Pro Cys Ile Asn Tyr Val Met Tyr Gly Gly Ala Gly Thr Pro Pro
    130                 135                 140

Val Pro Cys Cys Ser Gly Val Lys Gly Leu Tyr Lys Gln Ala Ser Ser
145                 150                 155                 160

Thr Ala Asp Arg Gln Ala Val Cys Gly Cys Leu Lys Thr Leu Ala Gly
                165                 170                 175

Thr Ala Ser Pro Ala Ile Leu Gly Thr Ala Ala Ala Leu Pro Gly Gln
            180                 185                 190

Cys Gly Val Ser Ile Pro Tyr Glu Ile Ser Pro Ser Thr Asp Cys Ser
        195                 200                 205

Lys Val Thr
    210

<210>  53
<211>  306
<212>  DNA
<213>  Artificial Sequence

<220>
<223>  MutB

<400>  53
atagaagaca tcatggcaat tggctgcggc actgtggtct cttatctgaa ctcatgcttg      60

ccctatgtga ccaacaaagg tcctttaggt ggttgctgcg gtggggtgaa gggactctat     120

ggagctgcac aaaccaccgc ggatcgtcag tccgtttgca gctgcctcaa atctcttgcc     180

agttcttaca aaggtgtcga tcttagcaag gcagccggac ttcctggtca atgtggtgtt     240

aacattccct ataaaatcag cccttccacc gattgctcaa aggtgaacta atcgagagtc     300

ttcata                                                                306

<210>  54
<211>  306
<212>  DNA
<213>  Artificial Sequence

<220>
<223>  MutD

<400>  54

atagaagaca tcatggcaat tggctgcggc actgtggtct cttatctgaa ctcatgccgc 60

ccctatgtga ccgacaaagg tcctttaggt ggttgctgca gtgggattaa gggactctat 120

aaggctgcac aaaccaccgc ggatcgtcag tccgtttgca gctgcctcaa aactcttgcc 180

gcttcttaca aaggtgtcga tcttggtaag gcagccggac ttcctcagca atgtggtgtt 240

aacattccct ataaaatcag cccttccacc gattgctcaa aggtgacgta atcgagagtc 300

ttcata 306

<210> 55
<211> 315
<212> DNA
<213> Artificial Sequence

<220>
<223> MutM

<400> 55
atagaagaca tcatggcatt atcttgcggc agcgtggctt ctgatctgtc cccgtgcatc 60

aactatgtga tgtatggtgg ggcggggacg ccaccggtac catgctgcag tggggtgaag 120

ggactctata agcaggcaaa atccaccgcg gatcgtcagg ccacttgcag ctgcctcaaa 180

actcttgcca gtaccgccag ccctgccatc cttagcactg cagccgctct tcctggtcaa 240

tgtggtgtta acattcccta taaaatcagc ccttccaccg attgctcaaa ggtgacgtaa 300

tcgagagtct tcata 315

<210> 56
<211> 315
<212> DNA
<213> Artificial Sequence

<220>
<223> MutN

<400> 56
atagaagaca tcatggcatt atcttgcggc agcgtggcta atgatctgtc cccgtgcatc 60

aactatgtga tgtatggtgg ggcggggacg ccaccggtac catgctgcag tggggtgaag 120

ggactctata agcaggcatc ttccaccgcg gatcgtcagg ccgtttgcgg atgcctcaaa 180

actcttgccg gtaccgccag ccctgccatc cttggtactg cagccgctct tcctggtcaa 240

tgtggtgttt ctattcccta tgaaatcagc ccttccaccg attgctcaaa ggtgacgtaa 300

tcgagagtct tcata 315

## Description

The present invention relates to a polypeptide comprising a sesame nsLTP (non-specific Lipid Transfer Protein) characterized by SEQ ID NO11 or a variant thereof, wherein the polypeptide is isolated, purified and/or immobilized, a diagnostically useful carrier comprising a solid phase coated with the polypeptide, a pharmaceutical composition comprising the polypeptide, a method comprising the step detecting in a sample the presence or absence of an antibody binding specifically to a sesame nsLTP characterized by SEQ ID NO11, a use of a polypeptide comprising a sesame nsLTP characterized by SEQ ID NO11 or a variant thereof, the polypeptide, carrier or kit for detecting an antibody binding specifically to a sesame nsLTP characterized by SEQ ID NO11 or for manufacturing a device for detecting said antibody, an enriched, isolated or purified antibody, preferably IgG or IgE class antibody, or a recombinant antibody binding specifically to a sesame nsLTP characterized by SEQ ID NO11 and a method for isolating such an antibody.

Allergic reactions to plants range from mild to systemic reactions, including severe anaphylactic reactions. Apart from allergic reactions upon consumption of recognizable plant products, allergens can be unrecognizable components in a variety of foodstuffs where their nature and hence the danger of allergic reactions is hidden.

In spite of the high prevalence of plant food allergy, few options are available for treatment, and avoidance is often the only possible option. Of course, this requires precise information regarding the nature of the antigen(s) to which the patient reacts.

A range of tests are commercially available, in particular regarding the most prevalent plant food components, in particular peanut, hazelnut and walnut. For example, Ara h 7 isotype 7.0201 has been identified as a major allergen in peanuts (US20180231567A1).

Such diagnostic tests for plant food allergies involve the detection of IgE and/or IgG antibodies from patients with a specificity with regard to proteins from an allergen source. However, a positive IgE test, i.e. IgE sensitization, does not always correlate with clinical manifestations. In other words, if IgE class antibodies to one allergen are detected, this observation is not sufficient to provide a conclusive diagnosis.

Rather, it is a first indication suggesting that the allergen source tested is more likely to be relevant than others. In clinical practice, a medical doctor's diagnosis of an allergy is usually based on a positive IgE or IgG sensitization for the relevant allergen source and a convincing clinical history of allergic reactions to this allergen. In addition, the results of complementary tests such as a skin prick test (SPT), performed by topical application of the specific extract on the skin of the patient, may be considered for the final diagnosis.

Following successful identification of an allergy to a specific allergen, a treatment may follow. While anti-histamines may be used to ameliorate treatment, a long-term and curative treatment can be performed with specific immunotherapy based on the controlled administration of the allergen. Although the exact mechanisms are not fully known, such a specific activation of the immune system alleviates the symptoms upon subsequent environmental exposure to the same allergen. Successful treatment has been demonstrated for various plant pollen and animal allergens. However, in some cases avoiding an allergen which has been identified as the causative agent of the allergy is the only feasible option.

Sesame allergy is an increasingly common plant food allergy. It is now estimated that at least 0.2% of children and adults are allergic to sesame, which approaches the prevalence rates of a number of well-known allergens such as soy and pistachio. It can cause severe allergic reactions with multiple organ system involvement (also known as anaphylaxis). As sesame is not listed as a major food allergen, consumers may be vulnerable to accidental exposure to this allergen. Sesame is present in a wide, and growing, variety of food products in the form of seeds, oils and pastes (i.e. tahini). Some cosmetics, medications, supplements and pet food also contain sesame.

A sesame allergy can be diagnosed by detecting specific IgE class antibodies based on sesame extracts or 2S albumin, Ses i 1, the major allergen. However, this leads to false negative results in around 30% of sesame allergic patients. Moreover, plant extracts are not the preferred source of antigens for tests, because they are hard to standardize, meaning that extracts prepared according to different protocols or based on different batches of raw materials yield different results. Moreover, minor antigens may be masked by other components or their concentration may be insufficient.

Therefore, the use of purified recombinant sesame allergens is preferred. However, there is a shortage of diagnostically useful sesame allergens. Oleosins, a class of major allergens in peanuts and hazelnuts, were shown to be only minor allergens in sesame allergy without diagnostic value (Ehlers, A.M., Rossnagel, M., Brix, B. et al. Sesame oleosins are minor allergens. Clin Transl Allergy 9, 32 (2019). https://doi.org/10.1186/s13601-019-0271-x).

There is a considerable need for additional diagnostic tools to minimize the need for food challenges.

Therefore, the problem underlying the present invention is to provide reagents and methods for the diagnosis or for aiding in the diagnosis of sesame allergy, a sesame LTP sensitization and/or an LTP syndrome.

Another problem underlying the present invention is to provide reagents and methods for the detection of sesame-specific antibodies, preferably IgG and IgE antibodies.

Another problem is to provide methods and reagents for the diagnosis of sesame allergy having an improved diagnostic reliability, in particular sensitivity and/or specificity, compared to state of the art methods and reagents.

Another problem underlying the present invention is to provide reagents and methods for the differential diagnosis or aiding in the differential diagnosis of sesame allergy, whereby a sesame allergy can be distinguished from other plant food allergies, in particular a peach allergy.

Another problem underlying the present invention is to identify or aid in identifying plant materials which should be avoided by a patient suffering from an allergy, preferably a plant food allergy, more preferably an LTP syndrome.

The problem underlying the present invention is solved by the subject-matter of the attached independent and dependent claims.

In a first aspect, the problem underlying the present invention is solved by a polypeptide comprising a sesame nsLTP characterized by SEQ ID NO11 or a variant thereof, wherein the polypeptide is isolated, purified and/or immobilized.

In a second aspect, the problem underlying the present invention is solved by a diagnostically useful carrier comprising a solid phase coated with a polypeptide according to the present invention.

In a preferred embodiment, the carrier is selected from the group comprising a bead, a test strip, a microtiter plate, a plastic surface, preferably polystyrene surface, a branched cellulose-based polymer, a microarray, a blot, preferably from the group comprising western blot, line blot and dot blot, a glass surface, a slide, a biochip and a membrane, and is most preferably a microtiter plate or a line blot.

In a preferred embodiment, the polypeptide is in complex with an antibody from the sample of a patient, preferably an IgG and/or IgE class antibody.

In a third aspect, the problem underlying the present invention is solved by a kit comprising
a) the carrier and a means for detecting an antibody bound to the carrier, which means is preferably a secondary antibody or a polypeptide comprising a sesame nsLTP characterized by SEQ ID NO11 or a variant thereof, or
b) a diagnostically useful carrier comprising a means for capturing an antibody in a liquid solution, preferably a non-labeled secondary antibody, and a means for detecting an antibody bound to the carrier, preferably a polypeptide comprising a sesame nsLTP characterized by SEQ ID NO11 or a variant thereof, more preferably with a detectable label,
wherein the kit preferably comprises, in addition to a) or b), one or more, preferably all reagents from the group comprising a calibrator, preferably a set of calibrators, a washing buffer, a positive control and a negative control.

In a preferred embodiment, the carrier is a test strip comprising spatially separate reagents which include a sesame nsLTP characterized by SEQ ID NO11 or a variant thereof, and one or more from the group comprising one or more calibrators, an anti-CCD (cross reactive carbohydrate determinants)-IgE control, a band or reagent indicating the presence of a secondary antibody, preferably a secondary antibody recognizing IgG and/or IgE class antibodies

In a fourth aspect, the problem underlying the present invention is solved by a pharmaceutical composition comprising a polypeptide comprising a sesame nsLTP characterized by SEQ ID NO11 or a variant thereof, preferably further comprising an adjuvant.

In a fifth aspect, the problem underlying the present invention is solved by a method comprising the step detecting in a sample the presence or absence of an antibody binding specifically to a sesame nsLTP characterized by SEQ ID NO11.

In a preferred embodiment, the sample is selected from the group comprising whole blood, serum and plasma, preferably from a mammal, more preferably human.

In a sixth aspect, the problem underlying the present invention is solved by a method comprising the step contacting a device comprising a solid phase coated with a polypeptide comprising a sesame nsLTP characterized by SEQ ID NO11 or a variant thereof with a buffered solution comprising an antibody binding specifically to a sesame nsLTP characterized by SEQ ID NO11, which solution is preferably not a sample from a patient in need of a diagnosis, wherein preferably
a) the concentration of the antibody in the solution is known, and/or
b) the antibody is a recombinant antibody and/or
c) the medical or diagnostic device is contacted with two or more solutions comprising the antibody, wherein the two or more solutions have a different concentration of the antibody, and/or
d) the antibody is recognized by secondary antibodies binding specifically to IgG and/or IgE class antibodies,
followed by detection of a signal which indicates whether or not the antibody has bound to the polypeptide, optionally a signal relating to the concentration of the antibody in the solution or solutions.

In a seventh aspect, the problem underlying the present invention is solved by a use of a polypeptide comprising a sesame nsLTP characterized by SEQ ID NO11 or a variant thereof, the polypeptide, carrier or kit according to the present invention for detecting an antibody binding specifically to a sesame nsLTP characterized by SEQ ID NO11 or for manufacturing a device for detecting said antibody.

In an eighth aspect, the problem underlying the present invention is solved by a use of a polypeptide comprising a sesame nsLTP characterized by SEQ ID NO11 or of an antibody binding specifically to a sesame nsLTP characterized by SEQ ID NO11 or the carrier according to the present invention for the manufacture of a diagnostic kit.

In a preferred embodiment, the antibody is an IgG or IgE class antibody and/or wherein the secondary antibody recognizes IgG and/or IgE class antibodies.

In a preferred embodiment, the antibody is detected using a method from the group comprising immunodiffusion, immunoelectrophoresis, light scattering, agglutination, labeled immunoassays, preferably from the group comprising radiolabeled immunoassays, enzyme immunoassays such as colorimetric assays, chemiluminescence immunoassays and immunofluorescence immunoassays.

In a ninth aspect, the problem underlying the present invention is solved by an enriched, isolated or purified antibody, preferably IgG or IgE class antibody, or a recombinant antibody binding specifically to a sesame nsLTP characterized by SEQ ID NO11.

In a tenth aspect, the problem underlying the present invention is solved by a method comprising the step enriching or isolating an antibody to a sesame nsLTP characterized by SEQ ID NO11.

The present invention is based on the inventors' surprising finding that antibodies exist in samples from sesame allergic patients which bind to polypeptides associated with the class of sesame nsLTP. The inventors have found that such antibodies do not exist in healthy patients and can be used to aid in the diagnosis of sesame allergy or an LTP syndrome.

According to the present invention a polypeptide comprising a sesame nsLTP characterized by SEQ ID NO11 or a variant thereof is provided. In a preferred embodiment, a sesame nsLTP is any polypeptide which is expressed by a plant from the order *Lamiales*, preferably *Sesamum sp*., more preferably *Sesamum indicum* and has a sequence which comprises SEQ ID NO11, preferably SEQ ID NO12, more preferably SEQ ID NO13 or a variant thereof. In a preferred embodiment, the polypeptide is selected from the group comprising SEQ ID NO1 (LTP1), SEQ ID NO2 (LTP2), SEQ ID NO3 (LTP3), SEQ ID NO4 (LTP4), SEQ ID NO5 (LTP5), preferably the group comprising SEQ ID NO2, SEQ ID NO3, SEQ ID NO4, SEQ ID NO5, most preferably the group comprising SEQ ID NO3, SEQ ID NO4, SEQ ID NO5 or a variant thereof. The inventors have found that the use of any of SEQ ID NO3 (LTP3), SEQ ID NO4 (LTP4), SEQ ID NO5 (LTP5) as an allergen in an immunoassay yields a stronger signal than SEQ ID NO2 (LTP2), but LTP2 still gives a stronger reaction than SEQ ID NO1 (LTP1), which yields a detectable, but weak signal only. In a preferred embodiment, the polypeptide comprises SEQ ID NO1 or a variant thereof. In a preferred embodiment, the polypeptide comprises SEQ ID NO2 or a variant thereof. In a preferred embodiment, the polypeptide comprises SEQ ID NO3 or a variant thereof. In a preferred embodiment, the polypeptide comprises SEQ ID NO4 or a variant thereof. In a preferred embodiment, the polypeptide comprises SEQ ID NO5 or a variant thereof.

The invention relates to a diagnostically useful carrier, which is preferably a solid phase carrier for contacting a means for specifically binding an antibody associated with said carrier, preferably a polypeptide comprising a sesame nsLTP characterized by SEQ ID NO11 or a variant thereof with a bodily fluid sample from a subject, preferably a mammalian subject, more preferably a human subject.

According to the present invention, the carrier comprises one or more means for specifically binding an antibody, preferably one or more, more preferably two or more, more preferably three or more such means, each of them capable of specifically capturing a different antibody, preferably from the group comprising an antibody to a polypeptide having SEQ ID NO1, an antibody to a polypeptide having SEQ ID NO2, an antibody to a polypeptide having SEQ ID NO3, an antibody to a polypeptide having SEQ ID NO4, an antibody to a polypeptide having SEQ ID NO5, an antibody to Pru p 3.0101 (P81402), an antibody to Pru p 3.0102 (Q9LED1), an antibody to Pru du 3.01 (C0L0I), an antibody to Mal d 3.0101w (Q5J026), an antibody to Mal d 3.0102w (Q5J011), an antibody to Mal d 3.0201w (Q5J009), an antibody to Mal d 3.0202w (Q5IZZ6), an antibody to Mal d 3.0203w (Q5IZZ5), an antibody to Jug r 3.0101 (C5H617), an antibody to Cor a 8.0101 (Q9ATH2), an antibody to Ara h 9.0101 (B6CEX8), an antibody to Ara h 9.0201 (B6CG41), an antibody to Art v 3.0101 (P0C088), an antibody to Art v 3.0201 (C4MGG9), an antibody to Art v 3.0202 (C4MGH0), an antibody to Art v 3.0301 (C4MGH1), an antibody to Tri a 14 (Q8GZB0), an antibody to Tri a 14.0201 (D2T2K2), an antibody to Par j 2.0101 (P55958), an antibody to Par j 2.0102 (O04403) and an antibody to Ole e 7.0101 (P81430). Throughout this application, any data base protein sequence code refers to the version available online at the earliest priority or filing date. Said one or more means is preferably immobilized on said carrier. In a preferred embodiment, the means for specifically binding an antibody is a polypeptide comprising or consisting of an antigen to which the antibody to be captured or detected binds specifically or a variant thereof. The polypeptide may be a linear peptide or a folded polypeptide, the latter preferably a variant adopting essentially the same fold as the native protein as may be determined by CD spectroscopy. In a preferred embodiment, the polypeptide comprises an epitope to the antibody to be captured or detected of at least 7, preferably 10, more preferably 15 amino acids. Said means, together with the insoluble carrier to which it is attached, may be separated from a reaction mixture, wherein it is contacted with a sample, in a straightforward manner, for example by filtration, application of a magnetic field, centrifugation or decanting.

In a preferred embodiment, the carrier is a line blot (Raoult, D., and Dasch, G. A. (1989), The line blot: an immunoassay for monoclonal and other antibodies. Its application to the serotyping of gram-negative bacteria. J. Immunol. Methods, 125 (1-2), 57-65; WO2013041540). In a preferred embodiment, the term "line blot", as used herein refers to a test strip, more preferably membrane-based, that has been coated with one or more means for specifically binding an antibody. If two or more means are used, they are preferably spatially separated on the carrier. Preferably, the width of the bands is at least 30, more preferably 40, 50, 60, 70 or 80% the width of the test strip. The line blot may comprise one or more control bands for confirming (a) that it has been contacted with sample sufficiently long and under adequate conditions, in particular in the presence of human serum, and/or (b) that essential reagents were present, in particular a secondary antibody and/or (c) a CCD control. A cut off band indicating whether a signal from another band is sufficiently strong to represent a positive result may also be included.

According to the present invention, a medical or diagnostic device such as the diagnostically useful carrier may be prepared by expressing a recombinant variant of a sesame nsLTP characterized by SEQ ID NO11 comprising an affinity tag, optionally with an artificial linker which may include a protease cleavage site, in a cell such as a eukaryotic or prokaryotic cell, contacting the expressed variant with a ligand binding specifically to the affinity tag, which ligand is immobilized on a solid phase, washing the solid phase such that non-specifically bound material from the cell is removed and eluting the expressed variant from the solid phase, preferably by adding an excess of non-immobilized ligand. The variant may then be immobilized on the device. Optionally, the affinity tag may be removed by contacting the variant with a protease, preferably a protease recognizing the protease cleavage site such as the commercially available prescission protease, before the immobilization. The affinity tag may be selected from the group of tags comprising His such as His6 or His8 or His 10, 18A, ACP, Aldehyd, Avi, BCCP, Calmodulin, Chitin binding protein, E-Tag, ELK16, FLAG, flash, poly glutamate, poly aspartate, GST, GFP, HA, Isope, maltose binding protein, myc, nus, NE, ProtA, ProtC, Tho1d4, S-Tag, SnoopTag, SpyTag, SofTag, Streptavidin, Strep-tag II, T7 Epitope Tag, TAP, TC, Thioredoxin, Ty, V5, VSV and Xpress Tag. Useful proteases include, but are not limited to TEV, Thrombin, Factor Xa or Enteropeptidase. Suitable linkers are part of vectors, for example pET vector series (Novagen).

In a preferred embodiment, a sesame nsLTP characterized by SEQ ID NO11 or a variant thereof is immobilized on a carrier. In a preferred embodiment, the term "coated", as used herein, means that the polypeptide is immobilized on the surface of a carrier. Any immobilization may be in a reversible or irreversible manner. For example, the immobilization is reversible if a polypeptide to be immobilized interacts with the carrier via ionic interactions which may be masked by addition of a high concentration of salt or if the polypeptide is bound via a cleavable covalent bond. By contrast, the immobilization is irreversible if such polypeptide is tethered to the carrier via a covalent bond that cannot be cleaved in aqueous solution under physiological conditions. For example, highly reactive esters such as N-hydroxysuccinimide may be used to immobilize a polypeptide irreversibly on a bead. The polypeptide may be indirectly immobilized, for example by immobilizing an antibody or other entity having affinity to the polypeptide, followed by addition of the polypeptide and formation of a polypeptide-antibody complex. The antibody captured may be detected using a labeled secondary antibody or a labeled means for specifically capturing the antibody to be detected. Methods for immobilizing are described in Wong, S. S., Chemistry of Protein Conjugation and Cross-Linking, 1991, CRC Press, and in Rosenberg, I. M., Protein Analysis and Purification, Second Edition, Springer, 2005, and in Thermo Scientific Pierce Antibody Production and Purification Technical Handbook, online version 2020.

In a preferred embodiment, a CCD control allows the identification of false positive results due to binding of IgE or similar antibodies to glycoprotein epitopes such as alpha-1,3-fucose on N-glycans (Altmann, F. (2016) Coping with cross-reactive carbohydrate determinants in allergy diagnosis, Allergo J Int 25(4), 98-105). This may be an additional line in a line blot or well in a microtiter plate, separate bead or other carrier or spatially separate part thereof comprising glycoprotein epitopes that, if negative, shows that there is no false negative reactivity in other bands. A multitude of line blots are commercially available, for example from EUROIMMUN Medizinische Labordiagnostika AG, Lübeck, Germany. In a preferred embodiment, the method according to the present invention comprises the step detecting the presence or absence of an antibody that binds to cross-reactive carbohydrate epitopes and so leads to false positive results. In another preferred embodiment, the method comprises treating a sample with CCD absorbent to remove such an antibody, and such an absorbent (ZD 3001-0101, EUROIMMUN) may be part of a kit according to the present invention.

In another preferred embodiment, the diagnostically useful carrier is a bead. Various beads for numerous applications are commercially available, mainly based on carbohydrate, for example sepharose or agarose, or plastic. They contain active or activatable chemical groups such as a carboxyl or ester group, which can be utilized for the immobilization of a means for specifically binding an antibody. Preferably, the beads are beads having an average diameter of from 0.1 µm to 10 µm, from 0.5 µm to 8 µm, from 0.75 µm to 7 µm or from 1 µm to 6 µm. The beads can be coated with the means for specifically capturing an antibody directly or *via* affinity ligands, for example biotin or glutathione and streptavidin and GST, respectively. For example, the bead may be coated with biotin or glutathione and the means for specifically binding an antibody or any polypeptide may be fused with streptavidin or glutathione-S-transferase or a variant thereof, respectively. Preferably, the bead is provided in the form of an aqueous suspension having a bead content of from 10 to 90%, preferably from 20 to 80%, preferably from 30 to 70%, more preferably from 40 to 60% (w/w). In a preferred embodiment, the bead is coated with a first detectable marker, for example a fluorescent dye. Chemiluminescence or fluorescence is a preferred method of detection if the carrier is a bead.

If more than one type of bead on which a distinct means for binding specifically an antibody to be detected is coated is used, each type may have a different type of detectable marker to make the types of beads distinguishable, for example for identifying the type of bead and bound antibody in flow cytometry. If the presence of an antibody binding specifically to a means is detected using a fluorescent label, the first detectable label, if also a fluorescent label, is chosen such that both can be distinguished, for example using separate lasers such as a green and a red laser for the detection of the label.

In a particularly preferred embodiment, the beads are paramagnetic beads, which can be easily concentrated on a surface with the aid of a magnet. For this purpose, commercial paramagnetic beads usually contain a paramagnetic mineral, for example iron oxide. A multiplicity of suitable paramagnetic beads is commercially available. A bead may be labeled with a detectable label.

In another preferred embodiment, the carrier is a hydrophilic branched polymer, preferably a cellulose polymer. Such polymers which may carry chemically reactive groups for the immobilization of antigenic polypeptides are commercially available, for example the ImmunoCAP from Phadia.

In another preferred embodiment, the carrier is a microtiter plate comprising at least 8 wells that may be used for ELISA. At least one of the wells is coated with the one or more means for specifically binding an antibody, either directly or indirectly.

According to the present invention, for ELISA, but also other assay formats, at least 2, 3, preferably 4, more preferably 5 calibrators are provided. In a preferred embodiment, a calibrator comprises an antibody binding specifically to said one or more means, at a defined concentration and may be used to set up a calibration curve for semi-quantitative analysis see Wild, The Immunoassay Handbook, 3rd Edition, 2005, Elsevier, Chapter 9. Preferably it is an antibody binding specifically to a sesame nsLTP characterized by SEQ ID NO11, but it could also be an antibody or similar reagent which binds specifically to the same means for capturing an antibody and/or the same means for detecting an antibody as the antibody to be detected does, preferably binding specifically to a sesame nsLTP characterized by SEQ ID NO11 and to a secondary antibody binding specifically to human IgE or IgG class antibodies. For example, a chimeric antibody comprising the same constant region as the antibody to be detected does, for example comprise the constant region of a human IgE antibody, while the variable region may be or may include parts from a recombinant antibody binding specifically to a sesame nsLTP characterized by SEQ ID NO11. See Hamilton RG. Application of engineered chimeric antibodies to the calibration of human antibody standards. Ann Biol Clin (Paris). 1991;49(4):242-8. PMID: 1928840. A set of calibrators comprises calibrators that comprise said antibody at known concentrations, which differ from each other. They are typically chosen such that they cover a significant part of the detection range For example, two calibrators may be provided at concentration ratios of at least 1:2, 1:5, 1:10, 1:50, 1:100, 1:200, 1:500 and 1:1000. A calibrator reflecting the absence of the antibody to be detected in a sample may also be included. When the inventive method is carried out, the calibrators may be processed and developed in parallel to the samples or before the samples are processed and developed. For some systems, a calibration curve can be stored and needs to be repeated only at periodic intervals.

In a preferred embodiment, a label is a chemical group which may be detected, preferably from the group comprising an enzymatically active label, for example a label having horse radish peroxidase activity or alkaline phosphatase activity, a chemiluminescent label, for example a molecule capable of generating a chemiluminescent signal such as an acridinium ester or luminol, a radioactive label, for example iodine 125, a spin label and a fluorescent label, for example FITC. Various labels have been described, for example by Wild, The Immunoassay Handbook, 3^{rd} Edition, 2005.

In another preferred embodiment, the carrier is a microarray. In a preferred embodiment, the term "microarray", as used herein, refers to a chip spotted with a variety of spatially separate allergenic polypeptides, among them one or more than one polypeptide according to the present invention or a variant thereof, preferably at least 5, 10, 20, preferably at least 30, 40, 50, 80 or 100.

In a preferred embodiment, the term "a means for detecting an antibody", as used herein, refers to a reagent that makes an antibody detectable which is part of a complex comprising an antigen such as a sesame nsLTP characterized by SEQ ID NO11 or a variant thereof immobilized on a carrier. In a preferred embodiment, the means is a molecule which binds specifically to all antibodies which have the same immunoglobulin class as the antibody to be detected, preferably IgE or IgG, more preferably a secondary antibody. In another preferred embodiment, the means is an antigen or variant thereof such as a sesame nsLTP characterized by SEQ ID NO11 or a variant thereof. It may bind to an antibody to be detected, which is immobilized such that not all of its antigen binding sites are blocked. For example, the antibody could be immobilized via its constant region or via one of its more than one binding sites on its variable regions. In a preferred embodiment, the means carries a detectable label.

In a preferred embodiment, a secondary antibody is an antibody binding specifically to all antibodies from an immunoglobulin class, preferably an immunoglobulin antibody class, more preferably a human immunoglobulin class such as IgG or IgE. Secondary antibodies typically recognize the constant domain of said class, but may also recognize other epitopes shared by antibodies from the class of interest, for example a conformational epitope across the 3D structure. A wide range of them is commercially available, for example from Thermo Fisher (such as Goat anti-Human IgE secondary antibody HRP, #A18793; Goat anti-Human IgG, Secondary antibody HRP, #H10307). It may be a monoclonal or a polyclonal antibody. In a preferred embodiment, the term "recognized", as used herein, means that the secondary antibody binds specifically to the antibody or antibodies to be detected. A secondary antibody may bind specifically to one or to more than one immunoglobulin class. This may be achieved by using as a secondary antibody to the class, preferably to IgG class antibodies, a mixture comprising an antibody binding specifically to each immunoglobulin or a single antibody which reacts with all immunoglobulin classes of interest. The use of secondary antibodies is explained in Kruger, N. J., Detection of Polypeptides on Blots Using Secondary Antibodies, in The Protein Protocols Handbook (ed. J. M. Walker), page 967, volume 1996, Springer. Briefly, such secondary antibodies may be generated by immunizing a laboratory animal with the antibody to be recognized or a mixture of the antibodies to be recognized.

In a preferred embodiment, it is determined whether at least one antibody binding to a sesame nsLTP characterized by SEQ ID NO11 or a variant thereof is present in sample, but the immunoglobulin class is not determined, *i.e.* it is not determined whether the one or more antibody is an IgG or IgE class is determined or if more than one antibody is present. Typically at least one means capable of binding specifically to a specific immunoglobulin class such as IgG or IgE is used. In a preferred embodiment, it is determined whether at least one antibody binding to at least allergen from a group comprising at least two allergens, among them a sesame nsLTP characterized by SEQ ID NO11 or a variant thereof, is present, but it is not determined to which allergen or allergens the at least one antibody binds. Typically at least a means capable of binding specifically to more than one specific immunoglobulin class such as IgG or IgE is used, for example a mixture of secondary antibodies, for example one binding to IgG and one binding to IgE, or a polypeptide comprising a sesame nsLTP characterized by SEQ ID NO11 or a variant thereof is used.

In a preferred embodiment, the term "a means for capturing an antibody", as used herein, is a means that may be used to immobilize an antibody on a solid phase, preferably all antibodies from an immunoglobulin class or an antibody binding specifically to a sesame nsLTP characterized by SEQ ID NO11 or a variant thereof. The means can be a secondary antibody, aptamer or other protein immobilized on the solid phase, which binds specifically to all antibodies having immunoglobulin class IgG and/or IgE. The means can be a sesame nsLTP characterized by SEQ ID NO11 or a variant thereof immobilized on the solid phase such that it only captures an antibody binding specifically to a sesame nsLTP characterized by SEQ ID NO11. Typically, the means does not carry a detectable label, by contrast to the means for detecting an antibody.

The sample from a subject preferably used to practice the present invention comprises antibodies, also referred to as immunoglobulins. Typically the sample is a bodily fluid comprising a representative set of the entirety of the subject's immunoglobulins. However, the sample, once provided, may be subjected to further processing which may include fractionation, centrifugation, enriching or isolating the entirety of immunoglobulins or any immunoglobulin class of the subject, preferably IgE and/or IgG, which may affect the relative distribution of immunoglobulins of the various classes. The sample may be selected from the group comprising whole-blood, serum, plasma, and saliva and is preferably serum. In a most preferred embodiment, the sample comprises IgE class antibodies. In a more preferred embodiment, the sample comprises a representative set of the subject's antibodies from classes IgA, IgG and IgE, preferably IgG and IgE, more preferably IgG1, IgG4 and IgE, wherein, most preferably, the ratio of antibodies to different antigens is essentially unaltered compared to the ratio in the sample as obtained from the subject.

The teachings of the present invention may not only be carried out using polypeptides having the exact sequences referred to in this application explicitly, for example by function, name, sequence or accession number, or implicitly, but also using variants of such polypeptides.

In a preferred embodiment, the term "variant", as used herein, may refer to at least one fragment of the full-length sequence referred to, more specifically one or more amino acid which is, relative to the full-length sequence, truncated at one or both termini by one or more amino acids. Such a fragment comprises or encodes a peptide having at least 6, 7, 8, 9, 10, 15, 25, 50, 60, 70, 80, 90 or 100 successive amino acids of the original sequence or a variant thereof. In a more preferred embodiment, the total length of the variant may be at least 25, 30, 40, 50, 60, 70, 80, 90, 100, 150, 200 or 250 or more amino acids. In another more preferred embodiment, the total length of the variant may be no more than 25, 30, 40, 50, 60, 70, 80, 90, 100, 150, 200 or 250 or more amino acids. SEQ ID NO45, SEQ ID NO46, SEQ ID NO47, SEQ ID NO48, SEQ ID NO49, SEQ ID NO50, SEQ ID NO51 and SEQ ID NO52 are examples of variants.

In another preferred embodiment, the term "variant" relates not only to at least one fragment, but also a polypeptide or a fragment thereof comprising amino acid sequences that are at least 40, 50, 60, 70, 75, 80, 85, 90, 92, 94, 95, 96, 97, 98 or 99% identical to the reference amino acid sequence referred to or the fragment thereof, wherein amino acids other than those essential for the biological activity, for example the ability to bind specifically to an antibody to be detected, or the fold or structure of the polypeptide are deleted or substituted and/or one or more such essential amino acids are replaced in a conservative manner and/or amino acids are added or deleted such that the biological activity of the polypeptide is at least partially preserved. The state of the art comprises various methods that may be used to align two given nucleic acid or amino acid sequences and to calculate the degree of identity, see for example Arthur Lesk (2008), Introduction to bioinformatics, Oxford University Press, 2008, 3rd edition. In a preferred embodiment, the ClustalW software (Larkin, M. A., Blackshields, G., Brown, N. P., Chenna, R., McGettigan, P. A., McWilliam, H., Valentin, F., Wallace, I. M., Wilm, A., Lopez, R., Thompson, J. D., Gibson, T. J., Higgins, D. G. (2007): Clustal W and Clustal X version 2.0. Bioinformatics, 23, 2947-2948) is used applying default settings.

In a preferred embodiment, variants may, in addition, comprise chemical modifications, for example labels such as isotopic labels or detectable labels or covalent modifications such as glycosylation, phosphorylation, acetylation, decarboxylation, citrullination, hydroxylation and the like. The person skilled in the art is familiar with methods for the modification of polypeptides. Moreover, variants may also be generated by way of fusion with other known polypeptides or variants thereof, for example artificial linkers, preferably not derived from a Sesame polypeptide, affinity tags, detection tag, a signal peptide, other antigens and the like. An artificial linker may comprise a sequence of amino acids which are not derived from the full-length sequence or wild type sequence, i.e. sesame nsLTP characterized by SEQ ID NO11, and may comprise 1 to 100, 2 to 50, 2 to 30, 2 to 20 or 2 to 10 amino acids. The linker may be flexible and comprise a high proportion, for example at least 50%, of soluble amino acids, preferably with short side chains such as glycine, alanine and serine. Other linkers may be rigid. The linker may comprise a protease cleavage site or its end may be the N- or C-terminal cleavage sequence obtainable after the cleavage. The person skilled in the art is familiar with linkers and protease cleavage sites and they are disclosed in the art, for example in Chen X, Zaro JL, Shen WC. Fusion protein linkers: property, design and functionality, Adv Drug Deliv Rev. 2013;65(10):1357-1369. A variant may be fused N-terminally and/or C-terminally with an artificial linker, followed by a detection tag or affinity tag at the N- and/or C-terminus. A signal peptide is a peptide which, when fused to a newly synthetized polypeptide, directs the latter to a certain location, for example the membrane, or leads to the secretion of the polypeptide into the medium useful for purification. Signal peptides are described in the state of the art, for example in Lin-Cereghino GP, Stark CM, Kim D, et al. The effect of α-mating factor secretion signal mutations on recombinant protein expression in Pichia pastoris. Gene. 2013;519(2):311-317. doi:10.1016/j.gene.2013.01.062, Freudl, R. Signal peptides for recombinant protein secretion in bacterial expression systems. Microb Cell Fact 17, 52 (2018), Owji, H. et al. (2018). A Comprehensive Review of Signal Peptides: Structure, Roles, and Applications. European Journal of Cell Biology. 97. 10.1016/j.ejcb.2018.06.003). Examples of variants comprising artificial linkers include SEQ ID NO49, SEQ ID NO50, SEQ ID NO51 and SEQ ID NO52.

The variant of the polypeptide has biological activity. In a preferred embodiment such biological activity is the ability to bind specifically to the respective antibody to be detected. In a preferred embodiment it comprises an epitope having the ability to bind specifically to the respective antibody, preferably from a sample from a patient suffering from a plant allergy, more preferably sesame allergy or an LTP syndrome.

When designing variants, the person skilled in the art may consider epitopes contributing to the biological activity. In a preferred embodiment, epitopes of other homologous allergens have been identified, in particular Pru p 3 and Par j 1 (Colombo et al, Identification of an Immunodominant IgE Epitope of the Parietaria judaica Major Allergen. The Journal of Immunology March 15, 1998, 160 (6) 2780-2785; Salcedo G et al, Plant non-specific lipid transfer proteins as food and pollen allergens. Clin Exp Allergy. 2004 Sep;34(9):1336-41) and this information may be used to identify reactive epitopes on the polypeptide according to the invention. In a preferred embodiment, epitopes of LTP1 represented by SEQ ID NO1 include peptides consisting of or comprising one or more sequences from the group comprising SGSQPPSAECCSKLKEQ (SEQ ID NO27) and AATCGVAFPTC (SEQ ID NO28). In a preferred embodiment, epitopes of SEQ ID NO2 include peptides consisting of or comprising one or more sequences from the group comprising VMYGGAGTPPVPCCTGIKTL (SEQ ID NO29), SSTADRQ (SEQ ID NO30) and PGKCGVSI (SEQ ID NO31). In a preferred embodiment, epitopes of SEQ ID NO3 include peptides consisting of or comprising one or more sequences from the group comprising VTDKGPLGGCCSGVKGL (SEQ ID NO32), KTTADRQ (SEQ ID NO33) and PQQCGVNI (SEQ ID NO34). In a preferred embodiment, epitopes of SEQ ID NO4 include peptides consisting of or comprising one or more sequences from the group comprising VTGTGALGGCCKGIKDL (SEQ ID NO35), QTTADRQ (SEQ ID NO36) and PSQCGLNI (SEQ ID NO37). In a preferred embodiment, epitopes of SEQ ID NO5 include peptides consisting of or comprising one or more sequences from the group comprising VTNKGPLGSCCGGVKGL (SEQ ID NO38), QTAQDRQ (SEQ ID NO39) and PGQCGVNI (SEQ ID NO40). In a preferred embodiment, the polypeptide according to the invention comprises one or more from the group comprising SEQ ID NO27, SEQ ID NO28, SEQ ID NO29, SEQ ID NO30, SEQ ID NO31, SEQ ID NO32, SEQ ID NO33, SEQ ID NO34, SEQ ID NO35, SEQ ID NO36, SEQ ID NO37, SEQ ID NO38, SEQ ID NO39 and SEQ ID NO40. The person skilled in the art may also consider that the inventors found that folded variants of a sesame nsLTP characterized by SEQ ID NO11 were more reactive than linear epitopes. Crucial cysteines in SEQ ID NO11 which are part of the disulphide bonds stabilizing overall structure should be conserved and preferably oxidized. Therefore, folded active portions based on fragments such as represented by SEQ ID NO41, SEQ ID NO42, SEQ ID NO43 and SEQ ID NO44 are a promising basis for designing variants.

In a preferred embodiment, a line blot assay comprising the variant as a means for specifically capturing an antibody, preferably as described in the examples, may be used to test whether the variant has such activity. The variant may also comprise N-terminal and/or C-terminal peptide or polypeptide fusions as long as these do not have specific binding activity to antigenic sequence parts and/or do not bind to other sequence parts, thus masking the antigenic sequence such that its binding characteristics are significantly altered and/or it is no longer capable of binding to the antibody.

The polypeptide may be provided in any form and at any degree of purification, from tissues, fluids or cells comprising said polypeptide in an endogenous form, more preferably cells overexpressing the polypeptide, crude or enriched lysates of such cells, to purified and/or isolated polypeptides which may be essentially pure. In a preferred embodiment, the polypeptide is a native polypeptide, wherein the term "native polypeptide", as used herein, refers to a folded polypeptide, more preferably to a folded polypeptide purified from cells, more preferably from prokaryotic or eukaryotic, preferably mammalian cells, more preferably plant cells. In another preferred embodiment, the polypeptide is purified from sesame or processed products made from sesame. A glycosylated form of the polypeptide may be used. In a preferred embodiment, the term "purified", as used herein, means that the polypeptide or any other purified reagent or molecule has been subjected to one or more method steps that effect a purification, preferably from the group comprising chromatography, more preferably from the group comprising size exclusion, affinity, hydrophobic interaction and ion exchange chromatography, more preferably affinity chromatography using an affinity tag such as a GST, MPB or His tag.

In a preferred embodiment, the polypeptide is used in a non-reduced state. This may be accomplished by purifying the polypeptide under non-reducing conditions or by oxidizing the polypeptide following the purification, for example by exposure to air in the absence of reducing agents such as DTT.

According to the present invention, the polypeptide may be a recombinant protein, wherein the term "recombinant", as used herein, refers to a polypeptide produced using genetic engineering approaches at any stage of the production process, for example by fusing a nucleic acid encoding the polypeptide to a strong promoter for overexpression in cells or tissues or by engineering the sequence of the polypeptide itself. The person skilled in the art is familiar with methods for engineering nucleic acids and polypeptides encoded (for example, described in Sambrook, J., Fritsch, E. F. and Maniatis, T. (1989), Molecular Cloning, CSH or in Brown T. A. (1986), Gene Cloning - an introduction, Chapman & Hall) and for producing and purifying native or recombinant polypeptides (for example Handbooks "Strategies for Protein Purification", "Antibody Purification", published by GE Healthcare Life Sciences, and in Burgess, R. R., Deutscher, M. P. (2009): Guide to Protein Purification). In another preferred embodiment, the polypeptide is an isolated polypeptide, wherein the term "isolated" means that the polypeptide has been enriched compared to its state upon production or as part of a natural raw material such as an unprocessed sample using a biotechnological or synthetic approach and is preferably pure, *i.e.* at least 60, 70, 80, 90, 95 or 99 percent of the polypeptide in the respective liquid consists of said polypeptide as judged by SDS polyacrylamide gel electrophoresis followed by Coomassie blue staining and visual inspection. Preferably any polypeptide on a carrier used as a means to capture an antibody is pure.

The inventive teachings provide a kit, preferably for diagnosing a plant food allergy, more preferably sesame allergy or an LTP syndrome. Such a kit is a container that comprises specific reagents required to practice the inventive method, in particular the diagnostically useful carrier according to the present invention, optionally in addition to one or more, preferably all reagents required to practice the inventive method. The kit may comprise a positive control, for example a recombinant antibody or antibody from a patient serum, preferably IgE or IgG, binding specifically to a sesame nsLTP characterized by SEQ ID NO11 or to the means used to bind specifically the antibody to be detected. For example, a polypeptide used as a means may comprise an artificial tag, and the antibody used as a positive control may bind specifically to said tag. The kit may comprise a negative control, for example a protein which does not bind specifically to an antibody binding specifically to a sesame nsLTP characterized by SEQ ID NO11, for example bovine serum albumin or a sample which does not comprise an antibody to binding specifically to a sesame nsLTP characterized by SEQ ID NO11, for example a blood sample such as a serum sample. The kit may comprise one or more calibrators. The kit may comprise a suitable water-tight vessel for contacting the diagnostically useful carrier with the sample in the presence of other liquids such as a reaction buffer. For example, a line blot may be provided in or in combination with an incubation tray, or a microtiter plate may be provided. Suitable vessels are described in the state of the art, for example EP3025780 or EP3025779.

According to the present invention, the specific binding between a sesame nsLTP, preferably characterized by SEQ ID NO11, more preferably selected from the group comprising LTP1, LTP2, LTP3, LTP4 and LTP5, and an antibody binding specifically to it and between other molecules, for example a secondary antibody and an antibody to be detected, is detected. In a preferred embodiment, the term "binding specifically", as used herein, means that the binding reaction is stronger than a binding reaction characterized by a dissociation constant of 1 × 10⁻⁵ M, more preferably 1 × 10⁻⁷ M, more preferably 1 × 10⁻⁸ M, more preferably 1 × 10⁻⁹ M, more preferably 1 × 10⁻¹⁰ M, more preferably 1 × 10⁻¹¹ M, more preferably 1 × 10⁻¹² M, as determined by surface plasmon resonance using Biacore equipment at 25 °C in PBS buffer at pH 7.

The methods or products according to the present invention may be used for diagnosing an allergy, preferably a plant food allergy, more preferably a sesame allergy. In a preferred embodiment, the allergy may be an or be associated with an LTP syndrome. In a preferred embodiment, the term "LTP syndrome", as used herein, refers to multiple plant-food sensitizations with a complex pattern of clinical manifestations such as anaphylaxis, associated with an allergy to at least one plant LTP. For background information, see EAACI Molecular Allergology User's Guide by Matricardi et al, Pediatr Allergy Immunol. 2016 May; 27 Suppl 23:1-250, and Asero, Eur Ann Allergy Clin Immunol . 2014 Jul;46(4):142-6. In another preferred embodiment, the allergy is characterized by a sensitization, preferably associated with clinical allergic symptoms.

In a preferred embodiment, the term "diagnosis", as used herein, is to be used in its broadest possible sense and may refer to any kind of procedure aiming to obtain information instrumental in the assessment whether a patient suffers or is likely or more likely than the average or a comparative subject, the latter preferably having similar symptoms, to suffer from a certain disease or disorder in the past, at the time of the diagnosis or in the future, to find out how the disease is progressing or is likely to progress in the future or to evaluate the responsiveness of a patient or patients in general with regard to a certain treatment, for example the administration of immunosuppressive drugs or an immunotherapeutic treatment, or to find out whether a sample is from such a patient. Such information may be used for a clinical diagnosis, but may also be obtained by an experimental and/or research laboratory for the purpose of general research, for example to determine the proportion of subjects suffering from the disease in a patient cohort or in a population. In other words, the term "diagnosis" comprises not only diagnosing, but also prognosticating and/or monitoring the course of a disease or disorder, including monitoring the response of one or more patients to the administration of a drug or candidate drug, for example to determine its efficacy. While the result may be assigned to a specific patient for clinical diagnostic applications and may be communicated to a medical doctor or institution treating said patient, this is not necessarily the case for other applications, for example in diagnostics for research purposes, where it may be sufficient to assign the results to a sample from an anonymized patient.

In a preferred embodiment, the methods and products according to the present invention may be used for interaction studies, including determining whether a drug candidate or other compound may interfere with the binding of an antibody to a sesame nsLTP characterized by SEQ ID NO11 or may affect any downstream process or the strength of their binding to their target. In a preferred embodiment, they may be used for monitoring the immune response, more preferably the emergence and/or titer of antibodies to a sesame nsLTP characterized by SEQ ID NO11, following the administration of an immunogenic composition comprising a sesame nsLTP characterized by SEQ ID NO11 or an immunogenic variant thereof, for example to a mammal, which may be a mammal other than a human such as a laboratory animal.

In many cases the mere detection of the antibody, in other words determining whether or not detectable levels of the antibody are present in the sample, is sufficient for aiding in the diagnosis. If the antibody can be detected, this information will be instrumental for the clinician's diagnosis and indicates an increased likelihood that the patient suffers from an allergy. The patient may then be subjected to additional tests, even invasive tests, such as a food challenge using sesame allergen or allergens, for example a sesame extract.

The person skilled in the art will appreciate that a clinician does usually not conclude whether or not the patient suffers or is likely to suffer from a disease, condition or disorders solely on the basis of a single diagnostic parameter, but needs to take into account other aspects, for example the presence of other antibodies, markers, blood parameters, clinical assessment of the patient's symptoms or the results of medical imaging or other non-invasive methods such as polysomnography, to arrive at a conclusive diagnosis. See Baenkler H. W. (2012), General aspects of autoimmune diagnostics, in Renz, H., Autoimmune diagnostics, 2012, de Gruyter, page 3. The value of a diagnostic agent or method may also reside in the possibility to rule out one disease, thus allowing for the indirect diagnosis of another. In a preferred embodiment, the meaning of any symptoms or diseases referred to throughout this application is in line with the person skilled in the art's understanding as of the filing date or, preferably, earliest priority date of this application as evidenced by text books and scientific publications. It should be mentioned that the inventive methods or uses or products, taken alone, cannot be used to arrive at a definite, final diagnosis.

In a preferred embodiment, the term "diagnosis" may also refer to a method or agent used to distinguish between two or more conditions associated with similar or identical symptoms, for example allergies to similar plants.

In a preferred embodiment, any information or data demonstrating the presence of absence of the autoantibody may be communicated to the patient or a medical doctor treating the patient orally, preferably by telephone, in a written form, preferably fax or letter, or in an electronic form *via* fax or *via* the internet, for example as an email or text message.

The present invention relates to a method comprising the step detecting in a liquid, for example a sample from a subject, the presence or absence of an antibody binding specifically to a sesame nsLTP characterized by SEQ ID NO11. Such a method may comprise the steps a) providing a liquid, preferably a patient sample, b) contacting the liquid with a carrier comprising one or more means for specifically binding the one or more antibodies to be detected under conditions compatible with the formation of a complex comprising the diagnostically useful carrier with the means and the antibody, more specifically the polypeptide comprising a sesame nsLTP characterized by SEQ ID NO11 or a variant thereof and the antibody, c) optionally isolating any said complex, for example by removing the liquid, d) optionally washing said complex, and e) detecting said complex. The method is preferably an *in vitro* method.

The detection of the antibody or complex for the prognosis, diagnosis, methods or kit according to the present invention comprises the use of a method selected from the group comprising immunodiffusion techniques, immunoelectrophoretic techniques, light scattering immunoassays, agglutination techniques, labeled immunoassays such as those from the group comprising radiolabeled immunoassays, enzyme immunoassays such as colorimetric assays, chemiluminscence immunoassays and immunofluorescence techniques. The person skilled in the art is familiar with these methods, which are also described in the state of the art, for example in Zane, H. D. (2001): Immunology - Theoretical & Practical Concepts in Laboratory Medicine, W. B. Saunders Company, in particular in Chapter 14. In a preferred embodiment, the term "presence" of an antibody, as used herein, means that the antibody is detectable using such a method, preferably a method selected from the group comprising enzyme immunoassays, more preferably colorimetric assays or chemiluminescence immunoassays, most preferably colorimetric assays based on line blot and detection using alkaline phosphatase activity as described in the examples. In a preferred embodiment, the term "absence", as used herein, means that the antibody is not detectable using such a method, owing to its absence or presence at extremely low concentrations below the respective detection limit.

In a preferred embodiment, two or more means for binding specifically an antibody to be detected are used. In a more preferred embodiment, it can be distinguished which antibody is present. This may be the case if the two or more means are spatially separated or antibodies are detected using distinguishable detection methods, for example labels comprising distinguishable fluorophores. In another preferred embodiment, it can only be determined that at least one of the two or more antibodies to be detected is present. This may be the case if a mixture of two or more means is used and it can only be determined that an antibody binds specifically to this mixture.

In many cases detecting, preferably meaning detecting the absence or presence of an antibody, optionally meaning determining whether the concentration of the antibody is above a certain threshold preferably as set by measurement using a detection method preferred according to this invention, for example ELISA or line blot, in the liquid, is sufficient for the diagnosis. If one or more antibody binding specifically to a sesame nsLTP characterized by SEQ ID NO11 can be detected, this will be information instrumental for the clinician's diagnosis and indicates an increased likelihood that the patient suffers from the allergy. The clinician will then be encouraged to follow up with more invasive tests such as oral food challenge or SPT.

In a preferred embodiment, the absence or presence of two or more antibodies is detected simultaneously, *i.e.* at the same time.

In a preferred embodiment, the absence or presence of two or more antibodies is detected in spatially separate reactions, more preferably in different reaction mixtures in separate vessels.

In a preferred embodiment, the present invention provides a use of a reagent or means or polypeptide for the detection of one or more antibodies binding specifically to a sesame nsLTP characterized by SEQ ID NO11, or of a nucleic acid encoding a sesame nsLTP characterized by SEQ ID NO11 or a variant thereof or a nucleic acid hybridizing specifically to said nucleic acid under stringent conditions or a vector or cell comprising said nucleic acid for the manufacture of a kit for the diagnosis of an allergy. In a preferred embodiment, a cell comprising said vector is provided and cultured under conditions allowing the expression of the nucleic acid, followed by isolating the expression product which may be a sesame nsLTP characterized by SEQ ID NO11 or a variant thereof, followed by use of the expression product as a means for specifically binding to an antibody in a method or product according to the present invention. More specifically, a diagnostically useful carrier may be coated with the expression product.

In another preferred embodiment, the method may be for confirming the reliability of an antibody detection assay or determining the concentration of an antibody binding specifically to a sesame nsLTP characterized by SEQ ID NO11 or a variant thereof and may involve detecting in a solution, which is not a sample from a patient, but is known or expected to comprise the antibody, preferably at a known concentration. Alternatively, the solution may be a negative control not comprising the antibody to check the background. Such method may be run in parallel with, after or before a diagnostic method.

In a preferred embodiment, the present invention provides an apparatus for analyzing a sample from a patient to detect one or more antibodies indicating an increased likelihood of an allergy, wherein the antibody is an antibody binding specifically to a sesame nsLTP characterized by SEQ ID NO11 or a variant thereof, comprising:
a) a carrier, which contains a means for capturing at least one antibody from the sample when the sample is contacted with the carrier,
b) a detectable means capable of binding to the antibody captured by the carrier when the detectable means is contacted with the carrier, particularly the detectable means is a labeled secondary antibody capable of binding to the antibody captured on the carrier,
c) optionally a means for removing any sample from the carrier and the detectable means, preferably by washing;
d) a detecting device for detecting the presence of the detectable means and converting the results into an electrical signal, and
e) optionally a means for receiving the electronical signal from the detecting device and determining if the level of the signal is indicative of an increased likelihood of a sesame allergy or LTP syndrome, by comparing with the level of signal detected in the background or an input reference value obtained with samples from healthy subjects.

The invention provides a pharmaceutical composition, preferably a vaccine, comprising one or more antigens selected from the group comprising a sesame nsLTP characterized by SEQ ID NO11 or a variant thereof, optionally in combination with one or more further plant antigens, which composition is preferably suitable for administration to a subject, preferably a mammalian subject, more preferably to a human. Such a pharmaceutical composition may comprise a pharmaceutically acceptable carrier. The pharmaceutical composition may, for example, be administered orally, parenterally, by inhalation spray, topically, by eyedrops, rectally, nasally, buccally, vaginally or via an implanted reservoir, wherein the term "parenterally", as used herein, comprises subcutaneous, intracutaneous, intravenous, intramuscular, intra-articular, intrasynovial, intrasternal, intrathecal, intralesional and intracranial injection or infusion techniques. The pharmaceutical composition may be provided in suitable dosage forms, for example capsules, tablets and aqueous suspensions and solutions, preferably in sterile form. It may be used in a method of treatment of a disease, preferably an allergy, which method comprises administering an effective amount of the inventive polypeptide to a subject. A hypoallergenic variant of the antigens may be used. The person skilled in the art is familiar with methods for the generation of hypoallergenic variants of known allergens. The pharmaceutical composition may be a sterilized liquid solution, which may be for injection. In a preferred embodiment, the pharmaceutical composition comprises an adjuvant, and at least one diluent, at least one glidant, at least one stabilizer and/or at least one filling agent. Suitable compositions are described in the state of the art, for example Plotkin, Orenstein and Offit, Vaccines, Elsevier Saunders, 2013 or Schijns, O'Hagan Immunopotentiators in Modern Vaccines, Elsevier Academic Press, 2006.

According to the present invention, a method comprising the step enriching or isolating an antibody to a sesame nsLTP characterized by SEQ ID NO11 is provided. The person skilled in the art is familiar with methods for enriching or isolating an antibody from various sources, and exemplary procedures are described in Thermo Scientific Pierce Antibody Production and Purification Technical Handbook, Thermo Scientific. Briefly, a source of the antibody, for example a sample from a patient comprising the antibody, preferably suffering from a disease such as sesame allergy or LTP syndrome, or from a laboratory animal immunized with a sesame nsLTP characterized by SEQ ID NO11 or a variant thereof, or a cell overexpressing a sesame nsLTP characterized by SEQ ID NO11 or a variant thereof or a lysate of such a cell is provided. The source of the antibody in the form of a liquid solution is then contacted with a sesame nsLTP characterized by SEQ ID NO11 or a variant thereof under conditions compatible with the formation of a complex comprising the sesame nsLTP and the antibody, followed by separation of the liquid sample from the complex. Typically, the sesame nsLTP is immobilized prior to or after formation of the complex on a solid phase which may be associated with any of the carriers disclosed herein as diagnostically useful carriers or any other solid phase, for example a chromatography column, followed by washing of the complex using a washing buffer. For example, the sesame nsLTP may be covalently linked with an agarose bead carrying an immobilized diamino dipropylamine linker via a side chain carboxy group using a crosslinker such as EDC. This is already an isolation of the antibody, but it may optionally be dissociated from the sesame nsLTP, for example by exposing the complex to denaturing conditions such as SDS in an electrophoretic purification step or a high salt buffer solution. Finally, the presence of the antibody, dissociated or not, may be detected by contacting it with a means for detecting an antibody. In a preferred embodiment, this method is used to prepare an antibody as a positive control, as a calibrator or for determining the concentration of the antibody in a solution, for example to prepare standardized solutions of the antibody as a control or calibrator.

According to the present invention, a polypeptide comprising a sesame nsLTP characterized by SEQ ID NO11 or an antibody binding specifically to a sesame nsLTP characterized by SEQ ID NO11 or the carrier according to the present invention may be used for the manufacture of a diagnostic kit, preferably for the diagnosis of an allergy, more preferably sesame allergy. For example, this may involve using the polypeptide and the antibody to coat a solid phase of a carrier, testing the quality of the carrier by performing a control reaction or calibrating with standardized components, for example a recombinant antibody rather than one from a patient sample, which may be mixed with a sample from a healthy blood donor, for example serum, packaging the components into a kit or actually performing steps of a method to obtain a result which may aid in the diagnosis.

According to the present invention, a vector comprising a nucleic acid encoding a sesame nsLTP characterized by SEQ ID NO11 or a variant is provided. Preferably, the nucleic acid is under the functional control of a promotor which allows for the constant or inducible expression of the polypeptide. Exemplary vectors include SEQ ID NO22, SEQ ID NO23, SEQ ID NO24, SEQ ID NO25, SEQ ID NO26 and variants thereof. According to the present invention, a cell, which may be prokaryotic or eukaryotic, comprising said vector is provided. The cell may be used to express the polypeptide, followed by purifying and immobilizing it on the solid phase of a device and use according to the present invention. The person skilled in the art is familiar with methods for the expression and purification of polypeptides using cells.

### Sequences:

The present invention comprises a range of novel nucleic acid and polypeptide sequences, more specifically the following, which are identical to sequences in the sequence listing. Solely as a precaution, any SEQ ID NO may refer both to sequences listed in this document and sequences in the sequence listing, in case of any unintended deviations.
**SEQ ID NO1 (*Sesamum indicum* lipid transfer protein LTP1, corresponds to EF581314.1)** 
MKKGAAAVWVVAIVVVLAVAEVHETGAAPCNPVELSPCLAAISGSQPPSAECCSKLKEQV
PCFCDYVKNPVFKPYVDSPNAKKVAATCGVAFPTC
**SEQ ID NO2 (EF581315.2, *Sesamum indicum* lipid transfer protein LTP2)** 
MEKATCLVLAVTMVVVALAPPQAEAAISCGTVANDLSPCINYVMYGGAGTPPVPCCTGIKTL
YNQASSTADRQAVCGCLKSVAGQASPAIIGTAAALPGKCGVSIPYEISPSTDCSKVH
**SEQ ID NO3 (EF581316.1, *Sesamum indicum* lipid transfer protein LTP3)** 
MAGTIKPMFVVLIAVVSIMSVVPEGEAAIGCGTVVSYLNSCFPYVTGTGALGGCCKGIKDLYA
AAQTTADRQSVCDCLKDLAASTTGVDLGKAAGLPSQCGLNIPYKIIPSTDCSKVQ
**SEQ ID NO4 (EF581317.1, *Sesamum indicum* lipid transfer protein LTP4)** 
MAGIIRPMCILLIAAVLAMAVAPRGEAAIGCGTVVSYLNPCLPYVTNKGPLGSCCGGVKGLY
GAAQTAQDRQSVCSCLKSLAGSYKDVDLNKAAGLPGQCGVNIPYKISPSTDCSKVN
**SEQ ID NO5 (EF581318.1, *Sesamum indicum* lipid transfer protein LTP5)** 
MASMIKALCVALVGAVLVIAVVPPAEAALGCGSVISYLGSCRPYVTDKGPLGGCCSGVKGLY
KAAKTTADRQATCSCLKTLASTYKGVNLSKAAGLPQQCGVNIPYKISPSTDCSKVT
**SEQ ID NO6 (*Sesamum indicum* lipid transfer protein (LTP1), cDNA fragment)** 
AGCGGTCTCACATGGCGCCTTGCAACCCGGTAGAGCTGTCGCCCTGCCTTGCAGCGAT
ATCAGGGTCGCAGCCGCCGTCGGCGGAGTGCTGCAGCAAGCTGAAGGAGCAGGTGCC
CTGCTTCTGTGACTACGTGAAGAACCCGGTTTTCAAGCCGTATGTCGACTCTCCTAATG
CCAAGAAGGTTGCTGCAACCTGCGGCGTCGCCTTCCCCACATGTTGACTCGAGGAGAC
CGAA
**SEQ ID NO7 (*Sesamum indicum* lipid transfer protein (LTP2), cDNA fragment)** 
AGCGAAGACATCATGGCCATATCATGCGGCACTGTGGCCAACGACCTGTCCCCGTGCA
TCAACTACGTGATGTATGGTGGGGCGGGGACGCCACCGGTACCATGCTGCACCGGGAT
 
AAAGACCCTCTACAATCAGGCAAGCAGCACCGCTGACCGCCAGGCTGTTTGTGGGTGC
TTGAAATCTGTTGCCGGCCAAGCCAGCCCTGCCATCATTGGCACAGCCGCCGCACTTC
CTGGCAAATGTGGCGTCTCTATTCCTTACGAGATCAGCCCCTCCACGGATTGCTCCAAG
GTTCATTAACTCGAGAGTCTTCGGT
**SEQ ID NO8 (*Sesamum indicum* lipid transfer protein (LTP3), cDNA fragment)** 
TTCGGTCTCACATGGCGATAGGTTGCGGCACTGTAGTTTCATATCTGAATTCATGCTTTC
CATACGTGACCGGAACTGGTGCGTTGGGTGGATGCTGTAAAGGGATTAAGGATCTCTAT
GCGGCGGCCCAAACCACAGCCGACCGACAGAGCGTGTGCGACTGCCTGAAAGATCTT
GCTGCCTCGACCACTGGCGTCGACCTCGGCAAAGCTGCTGGACTCCCCAGCCAGTGTG
GTCTTAACATCCCCTACAAGATCATTCCTTCAACTGATTGCTCAAAGGTGCAATGACTCG
AGGAGACCACC
**SEQ ID NO9 (*Sesamum indicum* lipid transfer protein (LTP4), cDNA fragment)** 
ACCGAAGACATCATGGCAATAGGCTGCGGCACGGTGGTTTCATACCTGAATCCATGCCT
GCCATATGTAACCAACAAAGGCCCTCTAGGCAGTTGTTGCGGTGGAGTTAAGGGTCTGT
ATGGAGCAGCCCAGACTGCACAAGATCGTCAAAGCGTGTGCAGCTGCTTGAAATCTCTC
GCTGGCTCATACAAAGATGTCGACCTGAACAAGGCCGCTGGACTCCCTGGACAATGTG
GCGTCAACATTCCCTACAAGATTAGCCCTTCCACTGATTGCTCAAAGGTGAATTGACTC
GAGAGTCTTCACC
**SEQ ID NO10 (*Sesamum indicum* lipid transfer protein (LTP5), cDNA fragment)** 
AGCGAAGACATCATGGCATTAGGCTGCGGCAGCGTGATATCTTATCTGGGTTCATGCCG
CCCCTATGTGACCGACAAAGGTCCTTTAGGTGGTTGCTGCAGTGGGGTGAAGGACTC
TATAAGGCTGCAAAAACCACCGCGGATCGTCAGGCCACTTGCAGCTGCCTCAAAACTCT
TGCCAGTACCTACAAAGGTGTCAATCTTAGCAAGGCAGCCGGACTTCCTCAGCAATGTG
GTGTTAACATTCCCTATAAAATCAGCCCTTCCACCGATTGCTCAAAGGTGACGTGACTC
GAGAGTCTTCACC
**SEQ ID NO11 (consensus sequence 1)** 
XXCGXXXXXLXXCXXXXXXXXXXXXXXXXCCXXXKXXXXXXXXXXXXXXXCXCXXXXXXXXXX
XXXXXXXXXXXXXXXCG
**SEQ ID NO12 (consensus sequence 2)** 
XXCGXVXXXLXXCXXYVXXXGXXXXXXXCCXGXKXLYXXAXXXXDRQXXCXCLKXXAXXX
XXXXXXXAAXLPXXCGXXIPYXIXPSTDCSKVX
**SEQ ID NO13 (consensus sequence 3)** 
XGCGXVXSYLXXCXPYVTXXGXLGXCCXGXKXLYXAAXTXXDRGXXCXCLKXLAXXXXXVX
LXKAAGLPXQCGXNIPYKIXPSTDCSKV
**SEQ ID NOS 14 TO 16 HAVE BEEN DELETED.**
**SEQ ID NO17 (α-factor-sp-H6-linker-PSc-Ses iLTP1)** 
MRFPSIFTAVLFAASSALAAPVNTTTEDETAQIPAEAVIGYSDLEGDFDVAVLPFSNSTNNGL
LFINTTIASIAAKEEGVSLEKRAMAHHHHHHGGGSGGGSGGGSLEVLFQGPSAMAPCNPVE
LSPCLAAISGSQPPSAECCSKLKEQVPCFCDYVKNPVFKPYVDSPNAKKVAATCGVAFPTC
**SEQ ID NO18 (α-factor-sp-H6-linker-PSc-Ses iLTP2)** 
MRFPSIFTAVLFAASSALAAPVNTTTEDETAQIPAEAVIGYSDLEGDFDVAVLPFSNSTNNGL
LFINTTIASIAAKEEGVSLEKRAMAHHHHHHGGGSGGGSGGGSLEVLFQGPSAMAISCGTV
ANDLSPCINYVMYGGAGTPPVPCCTGIKTLYNQASSTADRQAVCGCLKSVAGQASPAIIGTA
AALPGKCGVSIPYEISPSTDCSKVH
**SEQ ID NO19 (α-factor-sp-H6-linker-PSc-Ses iLTP3)** 
MRFPSIFTAVLFAASSALAAPVNTTTEDETAQIPAEAVIGYSDLEGDFDVAVLPFSNSTNNGL
LFINTTIASIAAKEEGVSLEKRAMAHHHHHHGGGSGGGSGGGSLEVLFQGPSAMAIGCGTV
VSYLNSCFPYVTGTGALGGCCKGIKDLYAAAQTTADRQSVCDCLKDLAASTTGVDLGKAAG
LPSQCGLNIPYKIIPSTDCSKVQ
**SEQ ID NO20 (α-factor-sp-H6-linker-PSc-Ses iLTP4)** 
MRFPSIFTAVLFAASSALAAPVNTTTEDETAQIPAEAVIGYSDLEGDFDVAVLPFSNSTNNGL
LFINTTIASIAAKEEGVSLEKRAMAHHHHHHGGGSGGGSGGGSLEVLFQGPSAMAIGCGTV
VSYLNPCLPYVTNKGPLGSCCGGVKGLYGAAQTAQDRQSVCSCLKSLAGSYKDVDLNKAA
GLPGQCGVNIPYKISPSTDCSKVN
**SEQ ID NO21 (α-factor-sp-H6-linker-PSc-Ses iLTP5)** 
MRFPSIFTAVLFAASSALAAPVNTTTEDETAQIPAEAVIGYSDLEGDFDVAVLPFSNSTNNGL
LFINTTIASIAAKEEGVSLEKRAMAHHHHHHGGGSGGGSGGGSLEVLFQGPSAMALGCGSV
ISYLGSCRPYVTDKGPLGGCCSGVKGLYKAAKTTADRQATCSCLKTLASTYKGVNLSKAAG
LPQQCGVNIPYKISPSTDCSKVT
**SEQ ID NO22 (pPICZd-sp-N-(PSc)-mSes iLTP1), SEQ ID NO23 (pPICZd-sp-N-(PSc)-mSes iLTP2), SEQ ID NO24 (pPICZd-sp-N-(PSc)-mSes iLTP3), SEQ ID NO25 (pPICZd-sp-N-(PSc)-mSes iLTP4) and SEQ ID NO26 (pPICZd-sp-N-(PSc)-mSes iLTP5)** are in the sequence listing only.
**SEQ ID NO27 to SEQ ID NO40 are disclosed in the text.**
**SEQ ID NO41 [fragment of LTP2]** 
CGTVANDLSPCINYVMYGGAGTPPVPCCTGIKTLYNQASSTADRQAVCGCLKSVAGQASP
AIIGTAAALPGKCG
**SEQ ID NO42 [fragment of LTP3]** 
CGTVVSYLNSCFPYVTGTGALGGCCKGIKDLYAAAQTTADRQSVCDCLKDLAASTTGVDLG
KAAGLPSQCG
**SEQ ID NO43 [fragment of LTP4]** 
CGTVVSYLNPCLPYVTNKGPLGSCCGGVKGLYGAAQTAQDRQSVCSCLKSLAGSYKDVDL
NKAAGLPGQCG
**SEQ ID NO44 [fragment of LTP5]** 
CGSVISYLGSCRPYVTDKGPLGGCCSGVKGLYKAAKTTADRQATCSCLKTLASTYKGVNLS
KAAGLPQQCG
**SEQ ID NO45 MutB** 
AIGCGTVVSYLNSCLPYVTNKGPLGGCCGGVKGLYGAAQTTADRQSVCSCLKSLASSYKG
VDLSKAAGLPGQCGVNIPYKISPSTDCSKVN
**SEQ ID NO46 MutD** 
AIGCGTVVSYLNSCRPYVTDKGPLGGCCSGIKGLYKAAQTTADRQSVCSCLKTLAASYKGV
DLGKAAGLPQQCGVNIPYKISPSTDCSKVT
**SEQ ID NO47 MutM** 
ALSCGSVASDLSPCINYVMYGGAGTPPVPCCSGVKGLYKQAKSTADRQATCSCLKTLASTA
SPAILSTAAALPGQCGVNIPYKISPSTDCSkVT
**SEQ ID NO48 MutN** 
ALSCGSVANDLSPCINYVMYGGAGTPPVPCCSGVKGLYKQASSTADRQAVCGCLKTLAGT
ASPAILGTAAALPGQCGVSIPYEISPSTDCSkVT
**SEQ ID NO49 α-factor-sp-H6-linker-PSc-MutB** 
MRFPSIFTAVLFAASSALAAPVNTTTEDETAQIPAEAVIGYSDLEGDFDVAVLPFSNSTNNGL
LFINTTIASIAAKEEGVSLEKRAMAHHHHHHGGGSGGGSGGGSLEVLFQGPSAMAIGCGTV
VSYLNSCLPYVTNKGPLGGCCGGVKGLYGAAQTTADRQSVCSCLKSLASSYKGVDLSKAA
GLPGQCGVNIPYKISPSTDCSKVN
**SEQ ID NO50 α-factor-sp-H6-linker-PSc-MutD** 
MRFPSIFTAVLFAASSALAAPVNTTTEDETAQIPAEAVIGYSDLEGDFDVAVLPFSNSTNNGL
LFINTTIASIAAKEEGVSLEKRAMAHHHHHHGGGSGGGSGGGSLEVLFQGPSAMAIGCGTV
VSYLNSCRPYVTDKGPLGGCCSGIKGLYKAAQTTADRQSVCSCLKTLAASYKGVDLGKAAG
LPQQCGVNIPYKISPSTDCSKVT
**SEQ ID NO51 α-factor-sp-H6-linker-PSc-MutM** 
MRFPSIFTAVLFAASSALAAPVNTTTEDETAQIPAEAVIGYSDLEGDFDVAVLPFSNSTNNGL
LFINTTIASIAAKEEGVSLEKRAMAHHHHHHGGGSGGGSGGGSLEVLFQGPSAMALSCGSV
ASDLSPCINYVMYGGAGTPPVPCCSGVKGLYKQAKSTADRQATCSCLKTLASTASPAILSTA
AALPGQCGVNIPYKISPSTDCSKVT
**SEQ ID NO52 α-factor-sp-H6-linker-PSc-MutN** 
MRFPSIFTAVLFAASSALAAPVNTTTEDETAQIPAEAVIGYSDLEGDFDVAVLPFSNSTNNGL
LFINTTIASIAAKEEGVSLEKRAMAHHHHHHGGGSGGGSGGGSLEVLFQGPSAMALSCGSV
ANDLSPCINYVMYGGAGTPPVPCCSGVKGLYKQASSTADRQAVCGCLKTLAGTASPAILGT
AAALPGQCGVSIPYEISPSTDCSKVT
**SEQ ID NO53 MutB** 
ATAGAAGACATCATGGCAATTGGCTGCGGCACTGTGGTCTCTTATCTGAACTCATGCTT
GCCCTATGTGACCAACAAAGGTCCTTTAGGTGGTTGCTGCGGTGGGGTGAAGGACTC
TATGGAGCTGCACAAACCACCGCGGATCGTCAGTCCGTTTGCAGCTGCCTCAAATCTCT
TGCCAGTTCTTACAAAGGTGTCGATCTTAGCAAGGCAGCCGGACTTCCTGGTCAATGTG
GTGTTAACATTCCCTATAAAATCAGCCCTTCCACCGATTGCTCAAAGGTGAACTAATCGA
GAGTCTTCATA
**SEQ ID NO54 MutD** 
ATAGAAGACATCATGGCAATTGGCTGCGGCACTGTGGTCTCTTATCTGAACTCATGCCG
CCCCTATGTGACCGACAAAGGTCCTTTAGGTGGTTGCTGCAGTGGGATTAAGGACTCT
ATAAGGCTGCACAAACCACCGCGGATCGTCAGTCCGTTTGCAGCTGCCTCAAAACTCTT
GCCGCTTCTTACAAAGGTGTCGATCTTGGTAAGGCAGCCGGACTTCCTCAGCAATGTGG
 
TGTTAACATTCCCTATAAAATCAGCCCTTCCACCGATTGCTCAAAGGTGACGTAATCGAG
AGTCTTCATA
**SEQ ID NO55 MutM** 
ATAGAAGACATCATGGCATTATCTTGCGGCAGCGTGGCTTCTGATCTGTCCCCGTGCAT
CAACTATGTGATGTATGGTGGGGCGGGACGCCACCGGTACCATGCTGCAGTGGGGT
GAAGGGACTCTATAAGCAGGCAAAATCCACCGCGGATCGTCAGGCCACTTGCAGCTGC
CTCAAAACTCTTGCCAGTACCGCCAGCCCTGCCATCCTTAGCACTGCAGCCGCTCTTCC
TGGTCAATGTGGTGTTAACATTCCCTATAAAATCAGCCCTTCCACCGATTGCTCAAAGGT
GACGTAATCGAGAGTCTTCATA
**SEQ ID NO56 MutN**

ATAGAAGACATCATGGCATTATCTTGCGGCAGCGTGGCTAATGATCTGTCCCCGTGCAT
CAACTATGTGATGTATGGTGGGGCGGGACGCCACCGGTACCATGCTGCAGTGGGGT
GAAGGGACTCTATAAGCAGGCATCTTCCACCGCGGATCGTCAGGCCGTTTGCGGATGC
CTCAAAACTCTTGCCGGTACCGCCAGCCCTGCCATCCTTGGTACTGCAGCCGCTCTTCC
TGGTCAATGTGGTGTTTCTATTCCCTATGAAATCAGCCCTTCCACCGATTGCTCAAAGGT
GACGTAATCGAGAGTCTTCATA

The present invention is further illustrated by the following non-limiting examples from which further features, embodiments, aspects and advantages of the present invention may be taken.
**Fig. 1** shows the isolated LTP proteins as in Example 1 following SDS-PAGE under reducing and non-reducing conditions followed by oriole fluorescent gel staining (r. reducing conditions; n. r. non-reducing conditions).
**Fig. 2** shows a EUROLINE line blot as used in Example 2.
**Fig. 3** shows the analysis of specific IgE binding of 146 sera from patients with LTP syndrome to LTP1 to 5 using the EUROLINE assay. The incubated strips were scanned and evaluated with the EUROLINEScan^{®} program, resulting in IgE reactivities expressed in kU/L.
**Fig. 4** shows the result of Western and Dot blotting as described in Example 3.

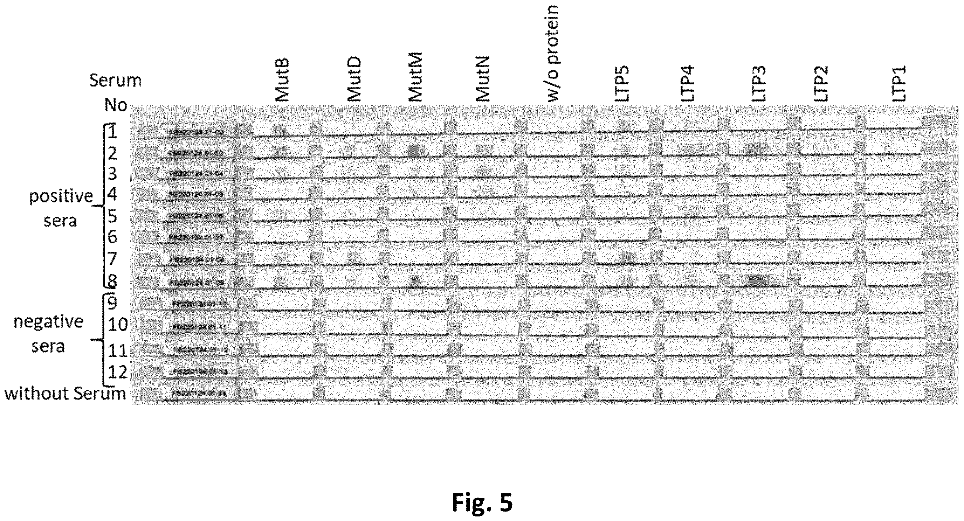

**Fig. 5** shows the results of Western blotting under non-reducing conditions of LTP1, LTP2, LTP3, LTP4 and LTP5 of *Sesamum indicum* and mutants MutB, MutD, MutM and MutN with 8 positive sera and four negative sera.

### Example 1: recombinant expression of Sesamum indicum lipid transfer proteins (LTP1 - LTP5) in Pichia pastoris:

### 1.1 Cloning of the lipid transfer proteins (LTPs)

Gene synthesis fragments coding for the lipid transfer proteins LTP1, LTP2, LTP3, LTP4 and LTP5 of *Sesamum indicum* (LTP1: Acc. EF581314.1, SEQ ID NO1; LTP2: Acc. EF581315.2, SEQ ID NO2; LTP3: Acc. EF581316.1, SEQ ID NO3; LTP4: Acc. EF581317.1, SEQ ID NO4; LTP5: Acc. EF581318.1, SEQ ID NO5) were obtained from Eurofins Genomics Germany GmbH (Ebersberg).

The gene synthesis products (SEQ ID NO6 to SEQ ID NO10 for LTP1, LTP2, LTP3, LTP4 and LTP5, respectively) were either digested with Bsal (New England Biolabs, R3733) or with Bbsl (New England Biolabs, R3539) and the fragments were gel-purified.

The gene synthesis fragments were ligated with Esp3I (New England Biolabs, R0734) into linearized adapted pPICZa vectors coding for lipid transfer protein (LTP1 - LTP5) of *Sesamum indicum* fused to the alpha-factor signal sequence and a N-terminal hexa histidine-tag (H6) (SEQ ID NO17 to SEQ ID NO21, respectively). Vectors represented by SEQ ID NO22, SEQ ID NO23, SEQ ID NO24, SEQ ID NO25, and SEQ ID NO26 were obtained and used to express LTP1, LTP2, LTP3, LTP4 and LTP5 each with the N-terminal His tag (SEQ ID NO17, SEQ ID NO18, SEQ ID NO19, SEQ ID NO20, SEQ ID NO21, respectively.

The coding sequences of the recombinant proteins include a linker-sequence and the sequence of the PreScission protease cleavage site between the histidine-tag and the sequences of the lipid transfer proteins of *Sesamum indicum.* The pPICZa-expression plasmids were all linearized by Sacl-digestion (New England Biolabs, R3156) for transformation of *Pichia pastoris* X-33, carried out according to the manual "pPICZ A, B, and C *Pichia* expression vectors for selection on Zeocin^{™} and purification of secreted, recombinant proteins", Cat. no. V195-20, Rev. Date: 7 July 2010, Manual part no. 25-0150, MAN0000035" by Invitrogen / Thermo Fisher Scientific.

### 1.2 Recombinant expression and purification of the lipid transfer proteins

The fusion proteins were expressed in *P. pastoris* as described by the manufacturer in the above manual from Cat. no. V195-20 and isolated by affinity chromatography on nickel matrix (IMAC) according to standard procedures.

### 1.3 Gel analyses and preparation of Western blots

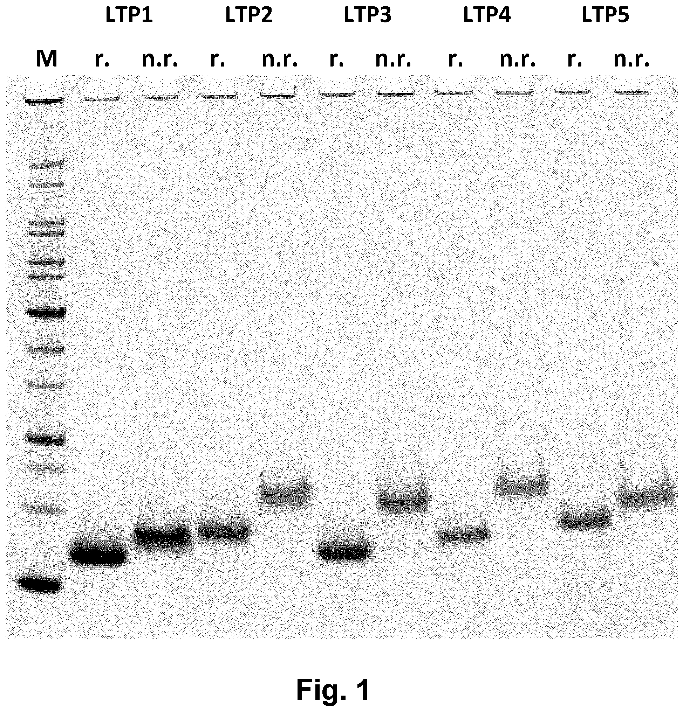

Purities of the isolated proteins were controlled by SDS-PAGE ((NuPage, 4 to 12% Gel, NuPAGE MES SDS running buffer, Thermo fisher) under reducing and non-reducing conditions followed by oriole fluorescent gel staining (BioRad) (Fig. 1) as recommended in the manufacturer's instructions.

For Western blot analyses proteins were separated by SDS-PAGE under reducing and non-reducing conditions and were blotted onto nitrocellulose membranes using the Fast Blotter system (Thermo Fisher) recommended in the manufacturer's instructions. The proteins were subsequently stained with Ponceau-S.

### Example 2: Line blot analysis of sesame LTP reactivity towards patient sera Preparation of a multiparameter line blot (EUROLINE)

### 2.1 Production of the EUROLINE strips

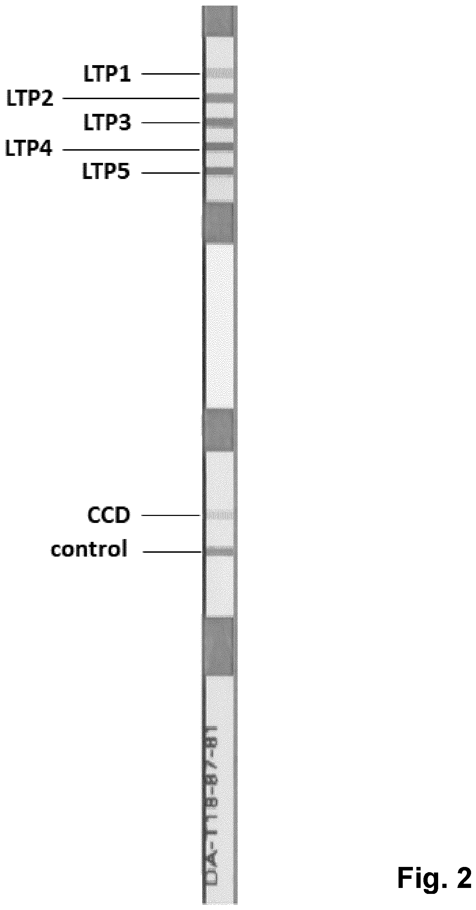

The EUROLINE line blot was produced by coating the isolated lipid transfer proteins LTP1, LTP2, LTP3, LTP4 and LTP5 as purified according to Example 1 using a precision dispenser (commercially available from Zeta Corporation) onto nitrocellulose membrane (commercially available from Whatman). After blocking and washing as recommended in the manufacturer's instructions the membranes containing the single proteins were aligned and cut in 2 mm strips, resulting in a multiparameter test. The final layout is shown in **Fig. 2**.

### 2.2 Evaluation of samples using the EUROLINE assay

The EUROLINE strips were incubated as recommended in the manufacturer's (EUROIMMUN Medizinische Labordiagnostika AG, Lübeck, Germany) instructions (Product number DP 3110-1601 E). In detail the strips were incubated with 1 ml 1/11 (v/v) diluted patient sample (146 sera from LTP syndrome patients) in washing buffer for 16 h at room temperature. After discarding the patient samples the strips were washed 3 times for 5 min with 1 ml washing buffer per strip and subsequently incubated for 1 h with 1 ml anti-human-IgE antibody conjugated with alkaline phosphatase. Afterwards the strips were again washed 5 times with washing buffer and then incubated for 10 min with BCIP/NBT substrate.

The evaluation of the results was carried out using a scanner and EUROLINEScan^{®} program (EUROIMMUN Medizinische Labordiagnostika AG, Lübeck, Germany). A semiquantitative analysis can be realized by using an IgE calibration curve of WHO standard (75/502).

### 2.3 Results

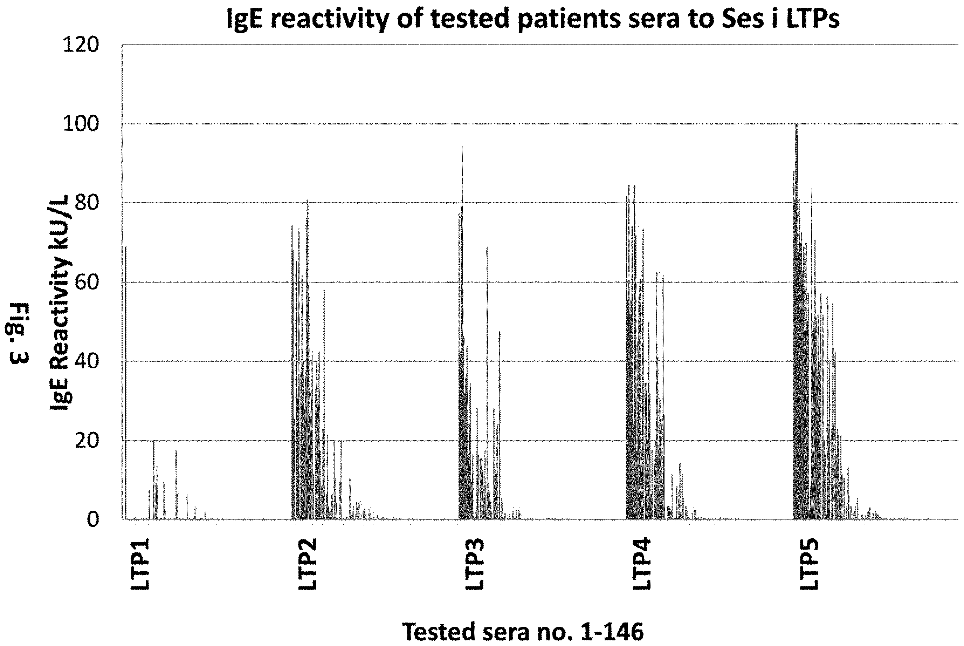

In the EUROLINE assay 146 sera from patients with LTP syndrome were tested (Fig. 3). Using the cut-off 0.35 kU/L for IgE reactivity, 17.8% of the sera showed positive IgE reactivity to LTP1, 56.2% to LTP2, 35.6% to LTP3, 43.8% to LTP4 and 55.5% to LTP5. In total 72.6% showed IgE reactivity to at least one tested protein.

The detection of specific IgE antibodies to one or more LTPs of sesame indicates an increased likelihood that the particular patient suffers from a sesame allergy and also from allergies to further LTPs from other sources, which then should be considered for the clinician's diagnosis.

### Example 3: Western and Dot blot analysis of sesame LTP reactivity towards patient sera

### 3.1 Western Blot and Dot Blot strips

For the dot blot strips 1 µL of each of the LTPs (LTP1, LTP2, LTP3, LTP4 and LTP5) as from Example 1 was dotted directly on nitrocellulose membrane (commercially available from Whatman). The membranes together with the Western blot membranes were cut in strips and stuck on transparent plastic sheets. The sheets were cut in strips again resulting in Western blot and dot blot strips each containing the 5 different Western blot and the dotted LTP membrane chips.

The strips were incubated separately overnight at room temperature with 400 µL patient serum respectively, that was diluted 1:10 (v/v) in universal buffer (product number ZD 1129-0101 E, EUROIMMUN Medizinische Labordiagnostika AG, Lübeck, Germany). After that the sera were discarded and the strips washed 3 × 5 min with 400 µL universal buffer. Then the strips were incubated at room temperature with 400 µL anti-human-IgE antibody conjugated with alkaline phosphatase for 1 hour. The strips were washed again 3 × 5 min with 400 µL universal buffer and then incubated with BCIP/NBT-substrate for 10 minutes until the reaction was stopped with demineralized water.

### 3.2 Results

Eight additional serum samples characterized as positive using the EUROLINE with the sesame LTPs 1 - 5 as described in Example 1, and two negative sera, which were negative with all sesame LTPs, were selected.

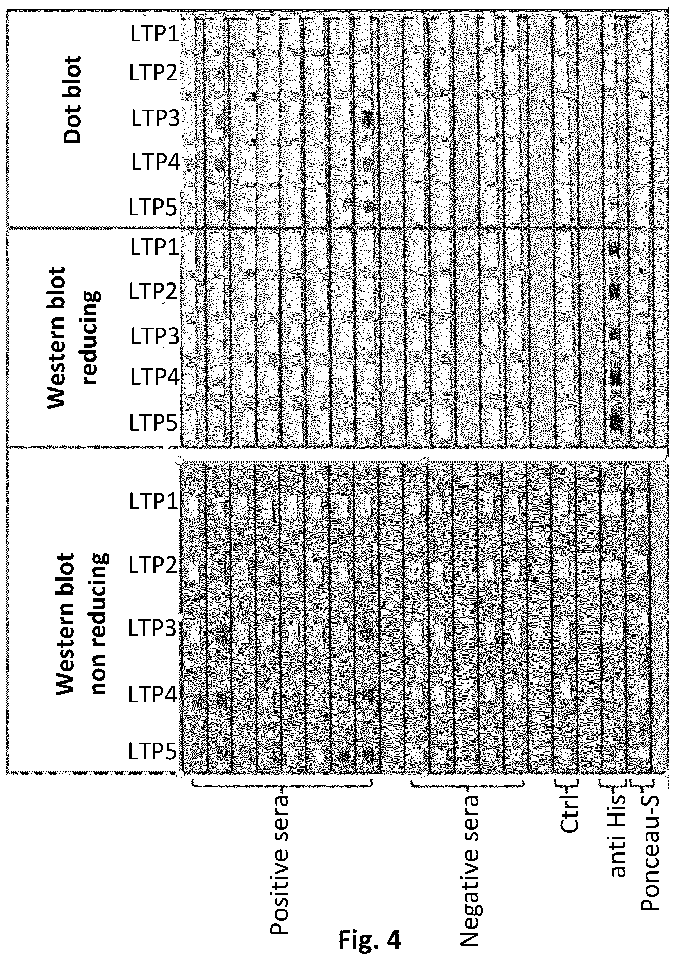

The positive sera showed strong reactions in a non-reducing Western blot against LTP3, LTP4 and LTP5 and weaker reactions with LTP1 and LTP2 (**Fig. 4**). Detection by dot blot yielded a similar result. Running the Western blot under reducing conditions reduced the reactivity considerably, suggesting that the protein folding is important for the antigen-antibody recognition. No reactions could be observed with either method if sera from subjects with a sesame LTP negative pre-characterization were examined.

### Example 3: Reactivity of positive sera with variants of sesame LTPs 1 - 5

### 4.1 Cloning of the Mutants of the lipid transfer proteins MutB, MutD, MutM and MutN

Gene synthesis fragments coding for mutants of the lipid transfer proteins of *Sesamum indicum* (Mutant B=SEQ ID NO53, Mutant D=SEQ ID NO54, Mutant M=SEQ ID NO55, Mutant N=SEQ ID NO56) were obtained from Eurofins Genomics Germany GmbH (Ebersberg). All mutants have sequence homology with sesame LTPs 1 - 5.

The gene synthesis products were either digested with Bsal (New England Biolabs, R3733) or with Bbsl (New England Biolabs, R3539) and the fragments were gel-purified.

The gene synthesis fragments were ligated with Esp3l (New England Biolabs, R0734) into linearized adapted pPICZa vectors coding for lipid transfer protein (MutB, MutD, MutM, MutN) of *Sesamum indicum* fused to the alpha-factor signal sequence and a N-terminal hexa histidine-tag (H6). Vectors were used to express MutB, MutD, MutM and MutN each with the N-terminal His tag (SEQ ID NO49, SEQ ID NO50, SEQ ID NO51, SEQ ID NO52, respectively).

The coding sequences of the recombinant proteins include a linker-sequence and the sequence of the PreScission protease cleavage site between the histidine-tag and the sequences of the Mutants of the lipid transfer proteins of *Sesamum indicum.* The pPICZa-expression plasmids were all linearized by Sacl-digestion (New England Biolabs, R3156) for transformation of *Pichia pastoris* X-33, carried out according to the manual "pPICZ A, B, and C Pichia expression vectors for selection on Zeocin™ and purification of secreted, recombinant proteins", Cat. no. V195-20, Rev. Date: 7 July 2010, Manual part no. 25-0150, MAN0000035" by Invitrogen / Thermo Fisher Scientific.

### 4.2 Recombinant expression and purification of the lipid transfer proteins

The fusion proteins were expressed in *P. pastoris* as described by the manufacturer in the above manual from Cat. no. V195-20 and isolated by affinity chromatography on nickel matrix (IMAC) according to standard procedures.

### 4.3 Preparation of Western blots

For Western blot analyses proteins the fusion proteins MutB, MutD, MutM and MutN and additionally LTP1, LTP2, LTP3, LTP4 and LTP5 from example 1 were separated by SDS-PAGE (NuPage, 4 to 12% Gel, NuPAGE MES SDS running buffer, Thermo fisher) under non-reducing conditions and were blotted onto nitrocellulose membranes using the Fast Blotter system (Thermo Fisher). The proteins were subsequently stained with Ponceau-S recommended in the manufacturer's instructions.

The western blot membranes were cut in strips and stuck on transparent plastic sheets. The sheets were cut in strips again resulting in strips each containing the 9 different western blots.

The strips were incubated separately 3 times for 5 min with 1 ml per strip 3 fold concentrated universal buffer (product number ZD 1129-0101 E, EUROIMMUN Medizinische Labordiagnostika AG, Lübeck, Germany). After discarding the liquid the strips were incubated separately overnight at room temperature with 400 µL patient serum (8 LPT positive and 4 negative sera) diluted 1:10 (v/v) in universal. After that the sera were discarded and the strips washed 3 × 5 min with 400 µL universal buffer. Then the strips were incubated at room temperature with 400 µL anti-human-IgE antibody conjugated with alkaline phosphatase for 1 hour. The strips were washed again 3 × 5 min with 400 µL universal buffer and then incubated with BCIP/NBT-substrate for 10 minutes until the reaction was stopped with demineralized water.

### 4.4 Results

Eight serum samples characterized as positive using the EUROLINE with the sesame LTPs 1 - 5 as described in Example 1 (of the Patent), and four negative sera, which were negative with all sesame LTPs, were selected.

The positive sera showed strong reactions in Western blot against LTP3, LTP4 and LTP5 as well as against the Mutants B, D, M and N and weaker reactions with LTP1 and LTP2 (**Fig. 1**).

No reactions could be observed if sera from subjects with a sesame LTP negative pre-characterization were examined.

Overall, it is shown that not only wild type sesame LTPs 1 - 5 may be used for carry out the teachings of the present invention, but also variants thereof.

## Claims

1. A polypeptide comprising a sesame nsLTP **characterized by** SEQ ID NO11 or a variant thereof, wherein the polypeptide is isolated, purified and/or immobilized.

2. A diagnostically useful carrier comprising a solid phase coated with the polypeptide according to claim 1.

3. The carrier according to claim 2, wherein the carrier is selected from the group comprising a bead, a test strip, a microtiter plate, a plastic surface, preferably polystyrene surface, a branched hydrophilic, preferably cellulose-based polymer, a microarray, a blot, preferably from the group comprising western blot, line blot and dot blot, a glass surface, a slide, a biochip and a membrane, and is most preferably a microtiter plate or a line blot.

4. The polypeptide or carrier according to any of claims 2 to 3, wherein the polypeptide is in complex with an antibody from the sample of a patient, preferably an IgG and/or IgE class antibody.

5. A kit comprising
a) the carrier according to any of claims 2 to 4 and a means for detecting an antibody bound to the carrier, which means is preferably a secondary antibody or a polypeptide comprising a sesame nsLTP **characterized by** SEQ ID NO11 or a variant thereof,
or
b) a diagnostically useful carrier comprising a means for capturing an antibody in a liquid solution, preferably a non-labeled secondary antibody, and a means for detecting an antibody bound to the carrier, preferably a polypeptide comprising a sesame nsLTP **characterized by** SEQ ID NO11 or a variant thereof, more preferably with a detectable label,
wherein the kit preferably comprises in addition to a) or b) one or more, preferably all reagents from the group comprising a calibrator, preferably a set of calibrators, a washing buffer, a positive control and a negative control.

6. The carrier or kit according to any of claims 1 to 5, wherein the carrier is a test strip comprising spatially separate reagents which include a sesame nsLTP **characterized by** SEQ ID NO11 or a variant thereof, and one or more from the group comprising one or more calibrators, an anti-CCD-IgE control, a reagent indicating the presence of a secondary antibody, preferably a secondary antibody recognizing IgG and/or IgE class antibodies.

7. A pharmaceutical composition comprising a polypeptide comprising a sesame nsLTP **characterized by** SEQ ID NO11 or a variant thereof, preferably further comprising an adjuvant.

8. A method comprising the step detecting in a sample the presence or absence of an antibody binding specifically to a sesame nsLTP **characterized by** SEQ ID NO11.

9. The method, wherein the sample is selected from the group comprising whole blood, serum and plasma, preferably from a mammal, more preferably human.

10. A method comprising the step contacting a device comprising a solid phase coated with a polypeptide comprising a sesame nsLTP **characterized by** SEQ ID NO11 or a variant thereof with a buffered solution comprising an antibody binding specifically to a sesame nsLTP **characterized by** SEQ ID NO11, which solution is preferably not a sample from a patient in need of a diagnosis, wherein preferably
a) the concentration of the antibody in the solution is known, and/or
b) the antibody is a recombinant antibody and/or
c) the medical or diagnostic device is contacted with two or more solutions comprising the antibody, wherein the two or more solutions have a different concentration of the antibody, and/or
d) the antibody is recognized by secondary antibodies binding specifically to IgG and/or IgE class antibodies,
followed by detection of a signal which indicates whether or not the antibody has bound to the polypeptide, optionally a signal relating to the concentration of the antibody in the solution or solutions.

11. A use of a polypeptide comprising a sesame nsLTP **characterized by** SEQ ID NO11 or a variant thereof, the polypeptide, carrier or kit according to any of claims 1 to 6 for detecting an antibody binding specifically to a sesame nsLTP **characterized by** SEQ ID NO11 or for manufacturing a device for detecting said antibody.

12. A use of a polypeptide comprising a sesame nsLTP **characterized by** SEQ ID NO11 or of an antibody binding specifically to a sesame nsLTP **characterized by** SEQ ID NO11 or the carrier according to any of claims 3, 4 or 6 for the manufacture of a diagnostic kit.

13. The carrier according to claim 4, the kit according to any of claims 5 to 6 or the method according to any of claims 8 to 10 or the use according to any of claims 11 to 12, wherein the antibody is an IgG or IgE class antibody and/or wherein the secondary antibody recognizes IgG and/or IgE class antibodies.

14. The method or use according to any of claims 8 to 12, wherein the antibody is detected using a method from the group comprising immunodiffusion, immunoelectrophoresis, light scattering, agglutination, labeled immunoassays, preferably from the group comprising radiolabeled immunoassays, enzyme immunoassays such as colorimetric assays, chemiluminescence immunoassays and immunofluorescence immunoassays.

15. An enriched, isolated or purified antibody, preferably IgG or IgE class antibody, or a recombinant antibody binding specifically to a sesame nsLTP **characterized by** SEQ ID NO11.

16. A method comprising the step enriching or isolating an antibody to a sesame nsLTP **characterized by** SEQ ID NO11.
